# EUROPÄISCHE PATENTSCHRIFT

(11) **EP 0 278 452 B1**
(45) Veröffentlichungstag und Bekanntmachung des Hinweises auf die Patenterteilung: **24.05.1995**
(21) Anmeldenummer: 88101791.7
(22) Anmeldetag: 08.02.1988
(51) Int. Cl.: C07D 213/79, C07D 213/80, C07D 213/81, C07D 405/12, A61K 31/44

(54) **Pyridin-2,4- und 2,5-dicarbonsäure-Derivate, Verfahren zu ihrer Herstellung, Verwendung derselben sowie Arzneimittel auf Basis dieser Verbindungen**
2,4- and 2,5-Pyridine-dicarboxylic-acid derivatives, process for their preparation, their use and medicines based on these compounds
Dérivés d'acide pyridine 2,4- et -2,5-dicarboxyliques, leur procédé de préparation, leur utilisation et médicaments à base de ces composés

(30) Priorität: 10.02.1987 DE 3703963
(43) Veröffentlichungstag der Anmeldung: 17.08.1988
(73) Patentinhaber: HOECHST AKTIENGESELLSCHAFT, 65926 Frankfurt am Main (DE)
(72) Erfinder: Bickel, Martin, Dr., D-6380 Bad Homburg (DE); Brocks, Dietrich, Dr., D-6200 Wiesbaden 42 (DE); Burghard, Harald, Dr., D-6384 Schmitten 3 (DE); Günzler, Volkmar, Dr., D-3550 Marburg-Cappel (DE); Henke, Stephan, Dr., D-6232 Bad Soden am Taunus (DE); Hanauske-Abel, Hartmut, Dr., D-6501 Dexheim (DE)

(56) Entgegenhaltungen:
- EP-A- 0 123 700
- EP-A- 0 176 741
- EP-A- 0 184 841
- EP-A- 0 203 435
- EP-A- 0 278 908
- US-A- 4 093 622
- JOURNAL OF THE AMERICAN SOCIETY, Band 97, 12. November 1975, Seiten 6662-6666; M.J.S. DEWAR et al.: "A thermodynamic study of the role of the central group on the stability of nematic liquid crystals"
- CHEMICAL ABSTRACTS, Band 71, 1969, Seite 367, Nr. 81100y, Columbus, Ohio, US; L. THUNUS et al.: "Preparation of monoesters of pyridinedicarboxylic acids", & J. PHARM. BELG. 1969, 24(1-2),3-21

## Beschreibung

Verbindungen, die die Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von der Zelle nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie α,α'-Dipyridyl zu einer Hemmung der C1_{q}-Biosynthese von Makrophagen führt (W. Müller et al., FEBS Lett. 90 (1978), 218; Immunbiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß Prolinhydroxylase durch Pyridin-2,4- und -2,5-dicarbonsäure effektiv gehemmt wird (K. Mayama et al., Eur. J. Biochem. 138 (1984) 239-245). Diese Verbindungen sind in der Zellkultur allerdings nur in sehr hohen Konzentrationen als Hemmstoffe wirksam (V. Günsler et al. Collagen and Rel. Research 3, 71 1983).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Überraschenderweise wurde nun gefunden, daß die gemischten Ester/Amide der Pyridin-2,4- und -2,5-dicarbonsäure und ebenso die höher alkylierten Diester ausgezeichnete Hemmstoffe der Kollagenbiosynthese im Tiermodell sind.

Der eigentliche Wirkstoff, die Pyridin-2,4- oder 2,5-Dicarbonsäure entsteht erst durch Hydrolyse der Ester oder Ester/Amide in der Zelle. Die Ester bzw. Ester/Amide können auf Grund ihrer höheren Lipophilie und der Tatsache, daß sie während des Transports überaschenderweise nur sehr langsam hydrolysiert werden bis in die Zellen transportiert werden. Erst hier wird der eigentliche Wirkstoff, Pyridin-2,4 oder -2,5-dicarbonsäure, freigesetzt.

Die Erfindung betrifft somit:
1. Pyridin-2,4- und -2,5-dicarbonsäure-Derivate der Formel I 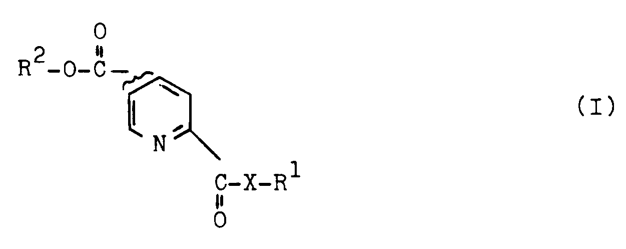 worin
   - R¹: verzweigtes oder unverzweigtes C₁-C₁₂-Alkyl, welches einfach oder im Falle der C₂-C₁₂-Alkyle auch mehrfach substituiert ist mit
   Halogen, Hydroxy, Cyano, Carboxyl, Alkoxy, Alkoxycarbonyl, Alkylcarbonyloxy, Alkyl- oder Dialkylamino, wobei die Alkylreste 1-4 C-Atome aufweisen, die im Falle der C₃- und C₄-Alkylreste auch verzweigt sein können,
   Phenyl, welches seinerseits gegebenenfalls mit Halogen, Nitro, C₁-C₄-Alkyl oder C₁-C₄-Alkoxy, 1-, 2-, oder 3-fach substituiert ist, wobei bei Mehrfachsubstitutionen die Substituenten auch voneinander unabhängig verschieden sein können und wobei im Falle der C₃- und C₄-Alkyle diese auch verzweigt sein können,
   oder R¹ gesättigtes C₅-C₇-Cycloalkyl, welches gegebenenfalls benzoanneliert ist,
   oder R¹ Phenyl, Naphthyl oder einen 5- oder 6-gliedrigen aromatischen Ring mit 1, 2 oder 3 Stickstoff- und/oder Sauerstoff- und/oder Schwefelatomen, welche gegebenenfalls ihrerseits mit Halogen, C₁-C₄-Alkyl oder C₁-C₄-Alkoxy 1-, 2-, oder 3-fach substituiert ist, wobei bei Mehrfachsubstitutionen die Substituenten auch voneinander unabhängig verschieden sein können und wobei im Falle der C₃-und C₄-Alkyle diese auch verzweigt sein können
   oder R¹ 2-Oxo-1,3-dioxolylmethyl, welches gegebenenfalls noch Methylsubstituiert ist
   oder R¹ wenn X Stickstoff ist, Wasserstoff
   und
   - R²: unabhängig von R¹ Wasserstoff oder R¹ ist, wobei R² auch identisch mit R¹ sein kann
   und
   - X: Sauerstoff oder mit R³ substituierter Stickstoff, wobei R³ Wasserstoff oder C₁-C₆-Alkyl ist und gegebenenfalls zusammen mit R¹ einen heterocyclischen, gesättigten 5-, 6- oder 7-Ring bildet, wobei der heterocyclische Ring auch ein zweites Stickstoffatom beinhalten kann und wobei der heterocyclische Ring seinerseits mit Phenyl oder Phenyl-C₁-C₃-Alkyl substituiert sein kann, bedeutet und deren physiologisch verträgliche Salze zur Anwendung als Arzneimittel.

Im Besonderen betrifft die Erfindung Pyridin-2,4- und -2,5-dicarbonsäure-Derivate gemäß Formel I, worin
- R¹: verzweigtes oder unverzweigtes C₁-C₄-Alkyl welches einfach oder im Falle der C₃- und C₄-Alkyle auch mehrfach substituiert ist, mit C₁-C₃-Alkoxy und/oder C₁-C₃-Alkoxycarbonyl, wobei die C₃-AlkylReste auch verzweigt sein können und/oder Phenyl
oder R¹ C₅- oder C₆-Cycloalkyl, das gegebenenfalls benzoanneliert ist,
oder R¹ Phenyl, das gegebenenfalls 1-, 2- oder 3-fach Nitrosubstituiert ist,
oder R¹ 2-, 3- oder 4-Pyridyl, Naphthyl, 2- oder 3-Thienyl, Pyrazolyl, Imidazolyl oder Thiazolyl
oder R¹ 5-Methyl-2-Oxo-1,3-dioxol-4-yl-methyl
bedeutet und
- R²: unabhängig von R¹ Wasserstoff oder R¹ ist, wobei R² auch identisch mit R¹ sein kann
und
- X: Sauerstoff oder mit R³ substituierter Stickstoff, wobei R³ Wasserstoff oder C₁-C₃-Alkyl ist und gegebenenfalls zusammen mit R¹ einen heterocyclischen gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann, bedeutet, sowie deren physiologisch verträgliche Salze, zur Anwendung als Arzneimittel.

Außerdem betriff die Erfindung Pyridin-2,4- und -2,5-dicarbonsäure-Derivate der Formel I', 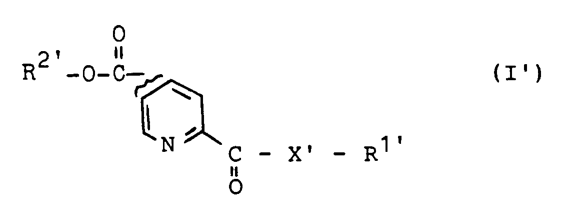
worin
- R^{1'}: verzweigtes oder unverzweigtes C₁-C₁₂-Alkyl, welches einfach oder im Falle von C₂-C₁₂-Alkyl auch mehrfach substituiert ist mit Halogen, Hydroxy, Cyano, Carboxyl, Alkoxy, Alkoxycarbonyl, Alkylcarbonyloxy, Alkyl- oder Dialkylamino, wobei die Alkylreste 1-4 C-Atome aufweisen, und wobei im Falle der C₃- und C₄Alkyle diese auch verzweigt sein können, Phenyl, welches seinerseits gegebenenfalls mit Halogen, C₁-C₄-Alkoxy oder C₁-C₄-Alkyl 1-, 2- oder 3-fach substituiert ist, wobei die genannten C₃- und C₄-Alkylreste auch verzweigt sein können und wobei bei Mehrfachsubstitutionen die Substituenten auch voneinander unabhängig verschieden sein können
oder R^{1'} gesättigtes C₅-C₇-Cycloalkyl, welches gegebenenfalls benzoanneliert ist
oder R^{1'} Phenyl, Naphthyl oder einen 5- oder 6-gliedrigen aromatischen Ring mit 1, 2 oder 3 Stickstoff- und/oder Sauerstoff- und/oder Schwefelatomen, welches gegebenenfalls seinerseits mit Halogen, Nitro, C₁-C₄-Alkyl oder C₁-C₄-Alkoxy 1-, 2- oder 3-fach substituiert ist, wobei bei Mehrfachsubstitutionen die Substituenten auch voneinander unabhängig verschieden sein können und wobei im Falle der C₃- und C₄-Alkyle diese auch verzweigt sein können,
oder R^{1'} 2-Oxo-1,3-dioxolylmethyl, welches gegebenenfalls Methyl-substituiert ist,
oder R^{1'} wenn X' Stickstoff ist, Wasserstoff,
und
- R^{2'}: unabhängig von R^{1'} Wasserstoff oder R^{1'} ist, wobei R^{2'} auch identisch mit R^{1'} sein kann
und
- X': Sauerstoff oder mit R^{3'} substituierter Stickstoff, wobei R^{3'} Wasserstoff oder C₁-C₆-Alkyl ist und gegebenenfalls zusammen mit R^{1'} einen heterocyclischen, gesättigten 5-, 6- oder 7-Ring bildet, wobei der heterocyclische Ring auch ein zweites Stickstoffatom beinhalten kann und wobei der heterocyclische Ring seinerseits mit Phenyl oder Phenyl-C₁-C₃-alkyl substituiert sein kann,
bedeutet,
sowie deren physiologisch verträgliche Salze,
ausgenommen die Verbindungen, in denen X' Sauerstoff bedeutet und R^{1'} und R^{2'} mit Chlor, Hydroxy oder Phenyl substituiertes Methyl oder Ethyl ist und Pyridin-2,5-dicarbonsäure-di(4-methoxy-phenyl)ester.

Bevorzugt sind Pyridin-2,4- und -2,5-dicarbonsäure-Derivate gemäß Formel I', worin
- R^{1'}: verzweigtes oder unverzweigtes C₁-C₄-Alkyl, welches einfach oder im Falle der C₂-C₄-Alkyle auch mehrfach substituiert ist mit Alkoxy, Alkoxycarbonyl, wobei die Alkylreste 1-3 C-Atome aufweisen und im Falle der C₃-Alkylverbindungen auch verzweigt sein können oder Phenyl
oder R^{1'} Cyclopentyl oder Cyclohexyl, die gegebenenfalls benzoanneliert sind
oder R^{1'} Phenyl, welches gegebenenfalls mit 1, 2 oder 3 Nitrogruppen substituiert ist oder Naphthyl
oder R^{1'} 2-, 3- oder 4-Pyridyl, 3-Thienyl, Pyrazolyl, Imidazolyl, Thiazolyl,
oder R^{1'} 5-Methyl-2-oxo-1,3-dioxol-4-yl-methyl
und
- R^{2'}: unabhängig von R^{1'}, Wasserstoff oder R^{1'} ist, wobei R^{2'} auch identisch mit R^{1'} sein kann
und
- X': Sauerstoff oder mit R^{3'} substituierter Stickstoff, wobei R^{3'} Wasserstoff oder C₁-C₃-Alkyl ist und gegebenenfalls zusammen mit R^{1'} einen heterocyclischen, gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
bedeutet,
sowie deren physiologisch verträgliche Salze,
ausgenommen die Verbindungen, in denen X' Sauerstoff bedeutet und R^{1'} und R^{2'} mit Chlor, Hydroxy oder Phenyl substituiertes Methyl oder Ethyl ist und Pyridin-2,5-dicarbonsäure-di(4-methoxy-phenyl)ester.

Besonders bevorzugt sind Pyridin-2,4- und -2,5-dicarbonsäure-Derivate der Formel I', worin R^{1'} verzweigtes oder unverzweigtes C₁-C₄-Alkyl, welches substituiert ist mit Alkoxycarbonyl, wobei die Alkylreste 1 - 3 C-Atome aufweisen, die im Falle der C₃-Alkylreste auch verzweigt sein können, und R^{2'} unabhängig von R^{1'} Wasserstoff oder R^{1'} ist, wobei R^{2'} auch identisch mit R^{1'} sein kann und
X' Sauerstoff bedeutet,
sowie deren physiologisch verträgliche Salze.

Diese Verbindungen besitzen u. a. eine besondere Wirksamkeit bei der oralen Applikation, wie auch die ganz besonders bevorzugten Pyridin-2,4- und -2,5-dicarbonsäure-Derivate der Formel I', worin R^{1'} und R^{2'} 1-Isopropoxycarbonylethyl-Gruppensind und X' Sauerstoff bedeutet (wie z. B. Pyridin-2,5-dicarbonsäure bis (1-isopropoxycarbonylethyl)ester (Bsp. 3) oder Pyridin-2,4-dicarbonsäure-bis(1-isopropoxycarbonylethyl)-ester (Bsp. 30)), sowie deren physiologisch verträgliche Salze.

Unter Halogen werden Fluor, Chlor, Brom und Jod, unter Aryl Phenyl und Naphthyl und unter Heteroaryl 5- und 6-gliedrige aromatische Ringe mit 1, 2 oder 3 Stickstoff- und/oder Sauerstoff- und/oder Schwefelatomen verstanden, die gegebenenfalls noch benzoanneliert sein können; insbesondere handelt es sich bei den Heteroarylresten um Pyridyl-, Pyridazyl-, Pyrimidyl-, Pyrazyl-, 1,3,5-Triazyl-, Pyrolyl-, Pyrazolyl-, Imidazolyl-, Triazolyl-, Thienyl-, Oxazolyl- und Thiazolyl-Reste und gegebenenfalls deren benzoannelierte Verbindungen.

"Mehrfach substituiert" bedeutet im Vorstehenden und Folgenden, daß mindetens 2 höchstens alle in den Alkylresten vorhandenen Wasserstoffatome durch die genannten Substituenten ersetzt sind. Bevorzugt handelt es sich dabei um einen Substituenten pro Methyl- bzw. Methylengruppe.

Bei Mehrfachsubstitutionen können die Substituenten auch voneinander unabhängig verschieden sein.

Weiterhin betrifft die Erfindung ein Verfahren zur Herstellung von Verbindungen der Formel I', das dadurch gekennzeichnet ist, daß man
a) eine Verbindung der Formel II 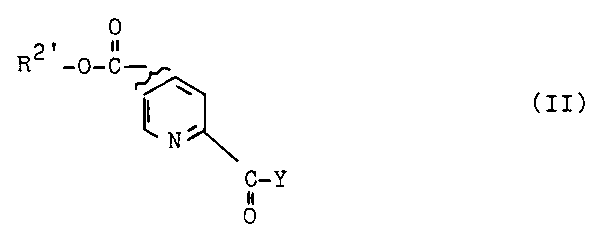 mit einer Verbindung der Formel III

   HX'-R^{1'} (III)

   umsetzt, wobei R^{1'}, R^{2'} und X' die zu Formel I' angegebenen Bedeutungen haben und Y Halogen oder Hydroxy ist oder
b) eine Verbindung der Formel IV 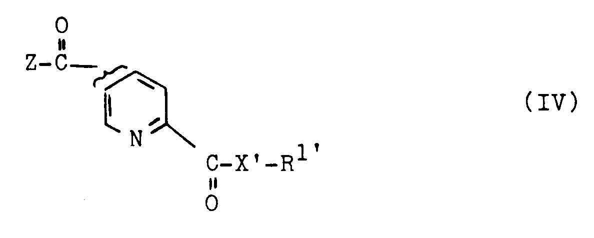 mit einer Verbindung der Formel V

   HO-R^{2'} (V)

   umsetzt, wobei R^{1'}, R^{2'} und X' die zu Formel I' angegebenen Bedeutungen haben und Z Halogen ist oder
c) eine Verbindung der Formel VI 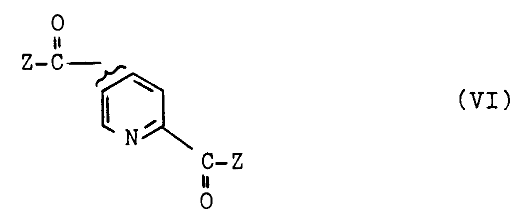 mit einem Alkohol HO-R^{2'} oder einem Alkohol der Formel VII

   HO-R^{1'} (VII)

   umsetzt, wobei R^{1'} und R^{2'} die zu Formel I' angegebenen Bedeutungen haben und Z Halogen ist oder
d) ein Alkalisalz der Pyridin-2,4- oder -2,5-dicarbonsäure mit einem Halogenid der Formel VIII

   R^{1'}-Z (VIII)

   unsetzt, wobei R^{1'} die zu Formel I' angegebenen Bedeutungen hat und Z Halogen ist
und die Reaktionsprodukte gegebenenfalls in ihre physiologisch verträglichen Salze überführt.

Im folgenden wird die Herstellung von Verbindungen gemäß Formel I und die Herstellung der dafür benötigten Ausgangssubstanzen - sofern sie nicht käuflich sind - näher beschrieben.

Die Herstellung der erfindungsgemäßen Verbindungen gelingt am einfachsten dadurch, daß die beiden Komponenten, das Pyridin-Derivat gemäß Formel (II), (IV) oder (VI) und das Amin bzw. der Alkohol gemäß Formel (III), (V) oder (VII) in äquimolaren Mengen oder bis zu einem etwa 5-fachen Überschuß an III, V oder VII vermengt werden und bei Temperaturen zwischen -30 bis 150 °C, bevorzugt bei 20 bis 100 °C bis zur Beendigung der Reaktion umgesetzt werden. Die Beendigung der Reaktion läßt sich mittels Dünnschichtchromatographie (DC-Kontrolle) bestimmen. Eine Variante dieses Verfahrens besteht darin, daß man in einem geeigneten Lösungsmittel, wie Diäthyläther oder Dimethoxyäthan oder Tetrahydrofuran, chlorierten Kohlenwasserstoffen wie Methylenchlorid, Chloroform, Tri- oder Tetrachloräthylen, Benzol, Toluol oder auch polaren Lösungsmitteln wie Dimethylformamid oder Aceton oder Dimethylsulfoxid arbeitet. Auch hier kann ein Überschuß von Amin/Alkohol gemäß Formel (III), (V) oder (VII), der bis zur etwa 5-fachen Mengen betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130°C besonders bevorzugt sind.

Gegebenenfalls kann die Umsetzung auch in Gegenwart von Basen erfolgen. Als zusätzliche Basen kommen anorganische Säurefänger wie Carbonate oder Hydrogencarbonate z.B. Natrium- oder Kaliumcarbonat oder Natrium- oder Kaliumhydrogencarbonat, oder organische Säurefänger wie tertiäre Amine, wie Triäthylamin, Tributylamin, Äthyldiisopropylamin oder heterocyclische Amine wie N-Alkylmorpholin, Pyridin, Chinolin oder Dialkylaniline in Betracht.

Bevorzugt erfolgt die Umsetzung der Verbindungen gemäß Formel (II) mit Alkoholen gemäß Formel (III) (X'= O) unter Zusatz eines wasserabspaltenden Mittels wie Dialkylcarbodiimid, wobei die Alkylreste 1 bis 8 C-Atome aufweisen, die im Falle der C₃-C₈-Verbindungen auch verzweigt oder cyclisch sein können; bevorzugt wird Dicyclohexylcarbodiimid eingesetzt. Eine entsprechende Methode ist in Houben-Weyl, Bd. XV/2, Seiten 103-111, Methoden der Organischen Chemie, 4. Aufl., Georg Thieme Verlag, Stuttgart, 1974, beschrieben.

Gegebenenfalls kann die Aufarbeitung der Produkte beispielsweise durch Extraktion oder durch Chromatographie z.B. über Kieselgel erfolgen. Das isolierte Produkte kann umkristallisiert und gegebenenfalls mit einer geeigneten Säure zu einem physiologisch verträglichen Salz umgesetzt werden. Als geeignete Säuren kommen beispielsweise in Betracht:
Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion-, Bernstein-Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (III), (V) und (VII) können, soweit sie nicht käuflich sind, einfach synthetisiert werden (z.B. Organikum, Organisch Chemisches Grundpraktikum, 15. Aufl., VEB Deutscher Verlag der Wissenschaften, 1976; eine Übersicht über die verschiedenen Möglichkeiten findet sich im Methodenregister; Alkohole: S. 821, Amine: S. 822).

Die Ausgangsverbindungen der Formel (II) erhält man beispielsweise durch Umsetzung von Pyridin-2,4- oder -2,5-dicarbonsäure zu dem entsprechenden Pyridin-2,4- oder -2,5-dicarbonsäurehalogenid (VI), bevorzugt -chlorid (nach literaturbekannten Verfahren, z.B. Organikum, Organisch Chemisches Grundpraktikum, 15. Aufl., VEB Deutscher Verlag der Wissenschaften, 1976, S. 595 ff), welches dann mit einem Alkohol der Formel R^{2'}-OH (V) zu dem entsprechenden 2,4- oder 2,5-Diester umgesetzt wird. Durch selektive Verseifung des Esters in der 2-Position des Pyridin-Derivats (z.B. über einen Kupferkomplex s. Pharm. Acta Helv. 44 1969, S. 637) oder partielle alkalische Verseifung (s. J. Org. Chem. 39 (8) 1974, S. 1158) erhält man die Pyridin-4 oder -5-Carbonsäureester-2-carbonsäure, die entweder direkt eingesetzt wird (II, Y=OH) oder in das Säurehalogenid (II, Y=Cl Br, J), bevorzugt das Säurechlorid überführt werden kann.

Die Ausgangsverbindungen der Formel (IV) können beispielsweise folgendermaßen synthetisiert werden:
Umsetzung des Pyridin-2,4- oder -2,5-Dicarbonsäurehalogenids, vorzugsweise des -chlorids, mit Benzylalkohol zum Pyridin-2,4- oder -2,5-dicarbonsäurebenzylester;
anschließende selektive Verseifung des Esters in 2-Position (z.B. in Gegenwart eines Kupferkatalysators, Loc.cit.Pharm. Acta Helv.), überführen der freien Säure in 2-Stellung in das Säurehalogenid, Umsetzung mit einer Verbindung der Formel HX'-R^{1'} (III) zum Pyridin-4- oder 5-Carbonsäurebenzylester-2-carbonsäure-(R^{1'})-Ester bzw. -amid, hydrogenolytischer Abspaltung der verbliebenen Benzylschutzgruppe (z.B. mit H₂/Pt, s. Houben-Weyl Bd. IV/1c (1980), S. 381-82) und anschließender Überführung der freien Säure in 4- bzw. 5-Position des Pyridinrings in das Säurehalogenid (IV).

Das Pyridin-2,4 oder -2,5-dicarbonsäurehalogenid gemäß Formel VI kann nach bekannten Methoden, z.B. durch Umsetzung von Pyridin-2,4- oder -2,5-dicarbonsäure mit Phosphortrihalogenid erhalten werden (s. z.B. Organikum, Organisch Chemisches Grundpraktikum, 15. Aufl., VEB Deutscher Verlag der Wissenschaften, 1976, S. 527 und 595ff).

Die Umsetzung von Alkalisalzen der Pyridin-2,4- oder -2,5-dicarbonsäure mit einem Halogenid der Fromel VIII erfolgt nach literaturbekannten Verfahren (s. z.B Organikum, Organisch Chemisches Grundpraktikum, 15. Auf., VEB Deutscher Verlag der Wissenschaften, 1976, S. 255 ff.)

Die erfindungsgemäßen Verbindungen der Formel I bzw. I' besitzen wertvolle pharmakologische Eigenschaften und zeigen insbesondere Wirksamkeit als Hemmer der Prolin- und Lysinhydroxylase, als Fibrosuppressivum und Immunsuppressivum.

Die Aktivität der Fibrogenase kann durch radioimmunologische Bestimmung des N-terminalen Propeptids des Kollagens Typ III oder der N- bzw. C-terminalen Quervernetzungsdomäne des Kollagens Typ IV (7s-Kollagen bzw. Typ-IV-Kollagen-NC₁) im Serum bestimmt werden.

Zu diesem Zweck wurden die Hydroxyprolin-, Prokollagen-III-Peptid-, 7s-Kollagen- und Typ-IV-Kollagen-NC₁-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, S. 335-476, New York, Academic Press, 1964).

Die pharmakologische Wirksamkeit der erfindungsgemäßen Substanzen wurde in einer Versuchsreihe (s. Tabelle 1) untersucht. Dabei zeigte sich eine deutliche Hemmung der Prolin- und Lysinhydroxylase.

**Tabelle 1**

| Substanz aus Bsp. | Dosierung | Hydroxyprolin »g/mg Leber | Prokollagen-III-Peptid ng/ml | 7s-Kollagen ng/ml | Typ-IV-Kollagen-NC1 ng/ml |
|---|---|---|---|---|---|
| 4 | 2 x 25 mg | 0.482 | 37.2 | 121.4 | 100.8 |
| CCl₄-Kontrolle | | 0.773 | 73.9 | 308.7 | 168.4 |
| Kontrolle | | 0.289 | 11.1 | 22.8 | 23.5 |

Die Verbindungen der Formel I bzw. I' können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Die pharmazeutischen Präparate können in fester Form, z.B. als Tabletten, Dragees, Suppositorien oder Kapseln; in halbfester Form, z.B. als Salben, oder in flüssiger Form, z.B. als Lösungen, Suspensionen oder Emulsionen, vorliegen. Gegebenenfalls sind sie sterilisiert und/oder enthalten Hilfsstoffe, wie Konservierungs-, Stabilisierungs-, Netz- oder Emulgiermittel, Salze zur Veränderung des osmotischen Drucks oder Puffer. Sie können auch noch andere therapeutisch wirksame Stoffe enthalten.

Im folgenden wird die Erfindung anhand von Beispielen näher erläutert:

### Beispiele

1. Pyridin-2,5-dicarbonsäure-bis(1-methoxycarbonylethyl)ester
   10 g Pyridin-2,5-dicarbonsäure werden in 60 ml trockenem Methylenchlorid vorgelegt und mit 80 ml frisch destilliertem Thionylchlorid und 2 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 12.5 g Milchsäuremethylester, gelöst in Methylenchlorid, bei -30 bis -20°C zugetropft. Man läßt die Mischung langsam auf Raumtemperatur erwärmen, rührt über Nacht bei Raumtemperatur und wäscht die Lösung mit Natriumbicarbonat-Lösung. Die organische Phase wird nach dem Trocknen vom Lösungsmittel befreit und über Kieselgel mit Essigester als Laufmittel chromatographiert. Das Produkt wird aus Isopropanol umkristallisiert.
   Schmelzpunkt 78°C; Ausbeute 7.2 g
2. Pyridin-2,5-dicarbonsäure-bis(1-ethoxycarbonylethyl)ester
   10 g Pyridin-2,5-dicarbonsäure werden in 60 ml trockenem Methylenchlorid vorgelegt und mit 80 ml frisch destilliertem Thionylchlorid und 2 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 14.1 g Milchsäureethylester gelöst in 1 l Methylenchlorid bei -30 bis -20°C zugetropft. Man läßt die Mischung langsam auf Raumtemperatur erwärmen, rührt über Nacht bei Raumtemperatur und wäscht die Lösung mit Natriumbicarbonat-Lösung. Die organische Phase wird nach dem Trocknen vom Lösungsmittel befreit und über Kieselgel mit Essigester als Laufmittel chromatographiert. Das Produkt fällt als Öl an.
   Ausbeute 16.6 g
3. Pyridin-2,5-dicarbonsäurebis(1-isopropoxycarbonylethyl)ester
   10 g Pyridin-2,5-dicarbonsäure werden in 60 ml trockenem Methylenchlorid vorgelegt und mit 80 ml frisch destilliertem Thionylchlorid und 2 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 15.8 g Milchsäureisopropylester gelöst in 100 ml Methylenchlorid bei -30 bis -20°C zugetropft. Man läßt die Mischung langsam auf Raumtemperatur erwärmen, rührt über Nacht bei Raumtemperatur und wäscht die Lösung mit Natriumbicarbonat-Lösung. Die organische Phase wird nach dem Trocknen vom Lösungsmittel befreit und mit Diisopropylether verrührt. Es wird vom Monoester abgetrennt und die Mutterlauge über Kieselgel mit einer Mischung von vier Teilen Toluol und einem Teil Essigester als Laufmittel chromatographiert. Das ölige Produkt kristallisiert langsam durch.
   Schmelzpunkt 52 - 53°C Ausbeute 13.5 g
4. Pyridin-2,5-dicarbonsäure-bis(2-methoxycarbonyl-2,2-dimethylethyl)ester
   10 g Pyridin-2,5-dicarbonsäure werden in 60 ml trockenem Methylenchlorid vorgelegt und mit 80 ml frisch destilliertem Thionylchlorid und 2 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 15.8 g 2,2-Dimethyl-3-hydroxypropionsäuremethylester gelöst in 100 ml Methylenchlorid bei -30 bis -20°C zugesetzt. Man läßt die Mischung langsam auf Raumtemperatur erwärmen, rührt über Nacht bei Raumtemperatur und wäscht die Lösung mit Natriumbicarbonat-Lösung.
   Die organische Phase wird nach dem Trocknen vom Lösungsmittel befreit und das Produkt aus Isopropanol umkristallisiert.
   Schmelzpunkt 114-5°C Ausbeute 18.6 g
5. Pyridin-2,4-dicarbonsäure-bis(2-methoxycarbonyl-2,2-dimethylethyl)ester
   7.5 g Pyridin-2,4-dicarbonsäure werden in 45 ml trockenem Methylenchlorid vorgelegt und mit 60 ml frisch destilliertem Thionylchlorid und 2 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 11.9 g 2,2-Dimethyl-3-hydroxypropionsäuremethylester gelöst in 100 ml Methylenchlorid bei -30 bis -20°C z ugesetzt. Man läßt die Mischung langsam auf Raumtemperatur erwärmen, rührt über Nach bei Raumtemperatur und wäscht die Lösung mit Natriumbicarbonat-Lösung.
   Die organische Phase wird nach dem Trocknen vom Lösungsmittel befreit und über Kieselgel mit Essigester als Laufmittel chromatographiert. Das Produkt fällt als Öl an. Ausbeute 6.7 g
6. Pyridin-2,4-dicarbonsäure-bis(1-ethoxycarbonylethyl)ester
   7.5 g Pyridin-2,4-dicarbonsäure werden in 45 ml trockenem Methylenchlorid vorgelegt und mit 60 ml frisch destilliertem Thionylchlorid und 2 ml trockenem Dimethylformamid versetzt. Die Mischung wird drei Stunden gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 10.6 g Milchsäureeethylester gelöst in 100 ml Methylenchlorid bei -30 bis -20°C zugetropft. Man läßt die Mischung langsam auf Raumtemperatur erwärmen, rührt über Nacht bei Raumtemperatur und wäscht die Lösung mit Natriumbicarbonat-Lösung. Die organische Phase wird nach dem Trocknen vom Lösungsmittel befreit und über Kieselgel mit Essigester als Laufmittel chromatographiert. Das Produkt fällt als Öl an, welches langsam durchkristallisiert.
   Schmelzpunkt 59 - 60°C Ausbeute 3.7 g.
7. Pyridin-2,4-dicarbonsäure-bis(5-methyl-2-oxo-1,3-dioxol-4-ylmethyl)ester
   6.3 g Pyridin-2,4-dicarbonsäure-Natriumsalz werden mit 14.3 g 5-Methyl-2-oxo-1,3-dioxol-4-yl-methylbromid und 4.5 g Kaliumcarbonat in 125 ml trockenem Aceton 40 Stunden unter Rückfluß gekocht. Die Lösung wird vom Carbonat abfriltriert und über Kieselgel mit einer 4:1 Mischung aus Toluol und Essigester chromatographiert.
   Schmelzpunkt 113°C Ausbeute 2.6 g
8. 2-(4-(2-Phenylethyl)piperazinocarbonyl)pyridin-5-carbonsäuremethylester
   1.5 g Pyridin-2-carbonsäure-5-carbonsäuremethylester werden mit 22.5 ml frisch destilliertem Thionylchlorid unter Rückfluß erhitzt bis eine klare Lösung entstanden ist. Man läßt eine Stunde bei Raumtemperatur nachrühren und destilliert das Thionylchlorid vollständig ab. Der Rückstand wird in 15 ml trockenem Methylenchlorid aufgenommen und zu einer Lösung von 3.15 g von 1-(2-Phenylethyl)-piperazin in 8 ml Methylenchlorid zugetropft, fünf Minuten bei Raumtemperatur nachgerührt und vom Lösungsmittel befreit. Der Rückstand wird unter Zusatz von wenig Aktivkohle aus Isopropanol umkristallisiert. Das Produkt fällt als Hydrochlorid an.
   Schmelzpunkt 201 - 203°C Ausbeute 2.6 g
9. 2-Benzylaminocarbonyl-pyridin-5-carbonsäuremethylester
   1.5 g Pyridin-2-carbonsäure-5-carbonsäuremethylester werden mit 22.5 ml frisch destilliertem Thionylchlorid unter Rückfluß erhitzt bis eine klare Lösung entstanden ist. Man läßt eine Stunde bei Raumtemperatur nachrühren und destilliert das Thionychlorid vollständig ab. Der Rückstand wird in 15 ml trockenem Methylenchlorid aufgenommen und zu einer Lösung von 1.15 g Benzylamin in 8 ml Methylenchlorid zugetropft, fünf Minuten bei Raumtemperatur nachgerührt und vom Lösungsmittel befreit. Der Rückstand wird unter Zusatz von wenig Aktivkohle aus Isopropanol umkristallisiert.
   Schmelzpunkt 215 - 216°C Ausbeute 1.9 g
10. 2-(N-Benzyl-N-methylaminocarbonyl)pyridin-5-carbonsäuremethylester
   1.5 g Pyridin-2-carbonsäure5-carbonsäuremethylester werden mit 22.5 ml frisch destilliertem Thionylchlorid unter Rückfluß erhitzt bis eine Klare Lösung entstanden ist. Man läßt eine Stunde bei Raumtemperatur nachrühren und destilliert das Thionylchlorid vollständig ab. Der Rückstand wird in 15 ml trockenem Methylenchlorid aufgenommen und zu einer Lösung von 2.0 g N-Methylbenzylamin in 8 ml Methylenchlorid zugetropft, fünf Minuten bei Raumtemperatur nachgerührt und vom Lösungsmittel befreit. Der Rückstand wird über Kieselgel mit einem Gemisch aus 7 Teilen Methylenchlorid und 3 Teilen Aceton chromatographiert.
   Das Produkt fällt als Öl an.
11. 2-Benzyloxycarbonylpyridin-5-carbonsäuremethylester
   1.5 g Pyridin-2-carbonsäure-5-carbonsäuremethylester werden mit 22.5 ml frisch destilliertem Thionylchlorid unter Rückfluß erhitzt bis eine klare Lösung entstanden ist. Man läßt eine Stunde bei Raumtemperatur nachrühren und destilliert das Thionylchlorid vollständig ab. Der Rückstand wird in 15 ml trockenen Methylenchlorid aufgenommen und zu einer Lösung von 1.79 g Benzylalkohol in 8 ml Methylenchlorid zugetropft, fünf Minuten bei Raumtemperatur nachgerührt und vom Lösungsmittel befreit. Der Rückstand wird unter Zusatz von wenig Aktivkohle aus Isopropanol umkristallisiert.
   Schmelzpunkt 104°C Ausbeute 1.5 g
12. 2-Phenylaminocarbonylpyridin-5-carbonsäuremethylester
   1.5 g Pyridin-2-carbonsäure-5-carbonsäuremethylester werden mit 22.5 ml frisch destilliertem Thionylchlorid unter Rückfluß erhitzt bis eine klare Lösung entstanden ist. Man läßt eine Stunde bei Raumtemperatur nachrühren und destilliert das Thionylchlorid vollständig ab. Der Rückstand wird in 15 ml trockenen Methylenchlorid aufgenommen und zu einer Lösung von 1.54 g Anilin in 8 ml Methylenchlorid zugetropft, fünf Minuten bei Raumtemperatur nachgerührt und vom Lösungsmittel befreit. Der Rückstand wird unter Zusatz von wenig Aktivkohle aus Isopropanol umkristallisiert.
   Schmelzpunkt 167°C Ausbeute 1.5 g
13. 2-(2,2-Diphenylethylamino)carbonylpyridin-5-carbonsäuremethylester
   1.5 g Pyridin-2-carbonsäure-5-carbonsäuremethylester werden mit 22.5 ml frisch destilliertem Thionylchlorid unter Rückfluß erhitzt bis eine klare Lösung entstanden ist. Man läßt eine Stunde bei Raumtemperatur nachrühren und destilliert das Thionylchlorid vollständig ab. Der Rückstand wird in 15 ml trockenen Methylenchlorid aufgenommen und zu einer Lösung von 3.27 g Diphenylethylamin in 8 ml Methylenchlorid zugetropft, fünf Minuten bei Raumtemperatur nachgerührt und vom Lösungsmittel befreit. Der Rückstand wird unter Zusatz von wenig Aktivkohle aus Isopropanol umkristallisiert.
   Schmelzpunkt 147°C Ausbeute 1.8 g
14. 2-(N-Methyl-N-Phenylamino)carbonylpyridin-5-carbonsäuremethylester
   25.5 g Pyridin-2-carbonsäure-5-carbonsäuremethylester werden mit 380 ml frisch destilliertem Thionylchlorid unter Rückfluß erhitzt bis eine klare Lösung entstanden ist. Man läßt eine Stunde bei Raumtemperatur nachrühren und destilliert das Thionylchlorid vollständig ab. Der Rückstand wird in 250 ml trockenen Methylenchlorid aufgenommen und zu einer Lösung von 30 g N-Methyl-anilin in 150 ml Methylenchlorid zugetropft, fünf Minuten bei Raumtemperatur nachgerührt und vom Lösungsmittel befreit. Der Rückstand wird unter Zusatz von wenig Aktivkohle aus Isopropanol umkristallisiert.
   Schmelzpunkt 123°C Ausbeute 30 g
15. 2-N-Propylamino-carbonylpyridin-5-carbonsäuremethylester
   15 g Pyridin-2-carbonsäure5-carbonsäuremethylester werden mit 225 ml frisch destilliertem Thionylchlorid unter Rückfluß erhitzt bis eine klare Lösung entstanden ist. Man läßt eine Stunde bei Raumtemperatur nachrühren und destilliert das Thionylchlorid vollständig ab. Der Rückstand wird in 70 ml trockenen Methylenchlorid aufgenommen und zu einer Lösung von 13.7 g Propylamin in 150 ml Methylenchlorid zugetropft. fünf Minuten bei Raumtemperatur nachgerührt und vom Lösungsmittel befreit. Der Rückstand wird über Kieselgel mit einer Mischung aus 7 Teilen Methylenchlorid und 3 Teilen Aceton als Laufmittel chromatographiert.
   Schmelzpunkt 88°C Ausbeute 12.6 g
16. Pyridin-2,5-di(4-nitro-phenyl)carbonsäureester
   16.7 g Pyridin-2,5-dicarbonsäure werden in 100 ml trockenem Methylenchlorid vorgelegt und mit 135 ml frisch destilliertem Thionylchlorid und 3 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine lösung von 27.8 g 4-Nitrophenol in 50 ml Pyridin bei -30 bis -20°C zugetropft. Man läßt die Mischung sich langsam auf Raumtemperatur erwärmen, rührt über Nacht bei Raumtemperatur und wäscht die Lösung mit Natriumbicarbonat-Lösung. Die organische Phase wird nach dem Trocknen vom Lösungsmittel befreit und über Kieselgel mit Essigester als Laufmittel chromatographiert.
   Schmelzpunkt 190 °C Ausbeute 12,5 g
17. Pyridin-2,5-dicarbonsäure-bis(5-methyl-2-oxo-1,3-dioxol-4-yl-methyl)ester
   6,3 g Pyridin-2,5-dicarbonsäure-Natriumsalz werden analog Beispiel 7 mit 14,3 g 5-Methyl-2-oxo-1,3-dioxol-4-yl-methylbromid umgesetzt und in Aceton 2,5 Stunden unter Rückfluß gekocht. Nach Chromatographie über Kieselgel mit Essigester als Laufmittel, wird das Produkt aus Essigester heiß umkristallisiert.
   Schmelzpunkt 118 °C Ausbeute 0,23 g
18. Pyridin-2,5-dicarbonsäure-di-(α-methoxycarbonylbenzyl)ester
   10 g Pyridin-2,5-dicarbonsäure werden analog Beispiel 1 in das Säurchlorid überführt und mit 19,9 g (±)-Mandelsäuremethylester umgesetzt. Die Aufarbeitung erfolgt nach der Extraktion durch Chromatographie über Kieselgel mit einer Mischung aus Toluol und Essigester als Laufmittel.
   Schmelzpunkt 125 °C Ausbeute 0,5 g
19. Pyridin-2-carbonamid-5-carbonsäuremethylester
   20 g Pyridin-2-carbonsäure-5-carbonsäuremethylester werden wie in Beispiel 8 beschrieben mit 200 g Thionylchlorid in das Säurechlorid überführt. Das Säurechlorid wird in Chloroform gelöst und es wird unter kräftigem Rühren Ammoniakgas über die Suspension gleitet. Man läßt zwei Tage stehen saugt das Produkt ab und wäscht mit Wasser.
   Schmelzpunkt 195-197 °C Ausbeute 15,6 g
20. Pyridin-2,5-dicarbonsäuredibenzylester
   20 g Pyridin-2,5-dicarbonsäure werden analog Beispiel 1 mit 160 ml Thionylchlorid ins Säurechlorid überführt und mit 25,9 g Benzylalkohol umgesetzt. Das Produkt wird aus Essigester unter Zusatz von Aktivkohle umkristallisiert.
   Schmelzpunkt 110°C Ausbeute 20,4 g
21. Pyridin-2-carbonsäure-5-carbonsäurebenzylester
   17 g Pyridin-2,5-dicarbonsäuredibenzylester werden in Methanol suspendiert und zu einer Suspension von 12,1 g Kupfer(II)nitrat gegeben. Es wird eine Stunde unter Rückfluß gekocht und nach dem Abkühlen vom Kupferkomplex abfiltriert. Der Komplex wird in Dioxan suspendiert und Schwefelwasserstoff bis zu einer Gewichtszunahme von 4 g eingeleitet. Es wird vom Kupfersulfid abgetrennt und die organsiche Phase eingeengt. Das Produkt wird aus Toluol umkristallisiert.
   Schmelzpunkt 132 °C Ausbeute 10,2 g
22. Pyridin-2-(3-isopropoxy-propyl)carbonsäureamid-5-carbonsäurebenzylester
   8 g Pyridin-2-carbonsäure-5-benzylester werden analog Beispiel 8 mit 90 ml Thionylchlorid in das Säurechlorid überführt und mit 3-Isopropyloxy-propylamin zum Amid umgesetzt. Das Produkt wird zur Reinigung über Kieselgel mit einer Mischung von Cyclohexan/Essigester (1:1) chromatographiert.
   Schmelzpunkt 41 °C Ausbeute 6,4 g
23. Pyridin-5-carbonsäure-2-(3-isopropoxy-propyl)carbonsäureamid
   5,3 g Pyridin-2-(3-isopropoxy-propyl)amid-5-benzylester werden fünf Stunden in Dioxan unter Normaldruck in Gegenwart eines Palladium-Kohle Katalysator hydriert. Nach Beendigung der Wasserstoffaufnahme wird vom Katalysator abgesaugt und vom Lösungsmittel abgezogen.
   Schmelzpunkt 129 °C Ausbeute 2,4 g
24. Pyridin-2-(3-isopropoxy-propyl)carbonsäureamid-5-carbonsäurebenzylester
   Entsprechend Beispiel 8 werden 1 g Pyridin-5-carbonsäure-2-(3-isopropoxy-propyl)amid in das Säurechlorid überführt (20 ml Thionylchlorid) und anschließen mit 2 ml Benzylalkohol zu dem Pyridin-2-isopropoxy-propylamid-5-benzylester umgesetzt. Das Produkt wird zur Reinigung über Kieselgel mit einer Mischung von Cyclohexan/Essigester (1:1) chromatographiert.
   Schmelzpunkt 41 °C Ausbeute 0,9 g
25. Pyridin-2,5-di(5-methyl-2-nitro-benzyl)carbonsäureester
   5 g Pyridin-2,5-dicarbonsäure werden analog Beispiel 1 mit 40 ml Thionylchlorid in 30 ml Methylenchlorid in das Säurechlorid überführt und mit 10 g 5-Methyl-2-nitro-benzylalkohol in 50 ml Methylenchlorid umgesetzt. Die Reaktionsmischung wird über Nacht bei Raumtemperatur gerührt und anschließend mit Natriumbicarbonatlösung versetzt, mit Methylenchlorid extrahiert und die organische Phase wird getrocknet. Nach Abziehen des Lösungsmittels wird der Rückstand mit Essigester verrührt, abgesaugt und zweimal aus Methylenchlorid umkristallisiert.
   Schmelzpunkt 182°C Ausbeute 2.9 g
26. Pyridin-2,5-dicarbonsäure-bis(2-ethoxy-ethyl)ester
   10 g Pyridin-2,5-dicarbonsäure werden analog Beispiel 1 mit 80 ml Thionylchlorid in 60 ml Methylenchlorid in das Säurechlorid überführt und mit 10.78 g Ethylenglykolmonomethylether in 100 ml Methylenchlorid umgesetzt. Die Aufarbeitung erfolgt durch Versetzen mit Natriumbicarbonatlösung, Abtrennen der organischen Phase und Abziehen des Lösungsmittels. Das Produkt wird zweimal über Kieselgel mit Essigester chromatographiert, in heißem Essigester gelöst, mit Aktivkohle geklärt und vom Lösungsmittel befreit. Das Produkt fällt als Öl an.
   Ausbeute 7.2 g
27. Pyridin-2,5-dicarbonsäure-bis(2-methoxy-ethyl)ester
   10 g Pyridin-2,5-dicarbonsäure werden analog Beispiel 26 ins Säurechlorid überführt und mit 9.1 g Ethylenglykolmonomethylether umgesetzt. Die Aufarbeitung erfolgt gemäß Beispiel 26. Das Produkt fällt als Öl an.
   Ausbeute 6.5 g
28. Pyridin-2,4-dicarbonsäure-bis(2-ethoxy-ethyl)ester
   10 g Pyridin-2,4-dicarbonsäure werden wie im Beispiel 26 beschrieben über das Säurechlorid mit Ethylenglykolmonoethylether umgesetzt. Die Reaktionsmischung wird analog Beispiel 26 aufgearbeitet. Das Produkt fällt als Öl an.
   Ausbeute 7.2 g
29. Pyridin-5-(2-methoxycarbonyl-2-methyl-propyl)carbonsäureester-2-N-(3-isopropoxy-propyl)carbonsäureamid
   1 g Pyridin-5-carbonsäure-2-(3-isopropoxy-propyl)amid-werden in 20 ml Thionylchlorid bis zur Lösung unter Rückfluß gekocht. Man läßt eine Stunde bei Raumtemperatur stehen, destilliert das Thionylchlorid ab, löst den Rückstand in 10 ml trockenem Methylenchlorid und tropft eine Lösung von 0.5 g 2,2-Dimethyl-3-hydroxypropionsäuremethylester in 20 ml trockenem Methylenchlorid zu. Nach Beendigung der Reaktion wird das Lösungsmittel abgezogen und das Produkt über Kieselgel mit Essigester chromatographiert.
   Ausbeute 0.15 g
30. Pyridin-2,4-dicarbonsäure-bis(1-isopropoxycarbonylethyl)ester
   10 g Pyridin-2,4-dicarbonsäure werden in 60 ml trockenem Methylenchlorid vorgelegt und mit 80 ml frisch destilliertem Thionylchlorid und 2 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 15.8 g Milchsäureisopropylester gelöst in 100 ml Methylenchlorid bei -30 bis -20°C zugetropft. Man läßt die Mischung langsam auf Raumtemperatur erwärmen, rührt über Nacht bei Raumtemperatur und wäscht die Lösung mit Natriumbicarbonat-Lösung. Die organische Phase wird nach dem Trocknen vom Lösungsmittel befreit und mit Diisopropylether verrührt. Es wird vom Monoester abgetrennt und die Mutterlauge über Kieselgel mit einer Mischung von vier Teilen Toluol und einem Teil Essigester als Laufmittel chromatographiert. Das Produkt fällt als Öl an.
   Ausbeute: 11,2 g

## Patentansprüche (Patentansprüche für folgende(n) Vertragsstaat(en): AT, BE, CH, DE, FR, GB, IT, LI, LU, NL, SE)

1. Pyridin-2,4- und -2,5-dicarbonsäure-Derivate der Formel I 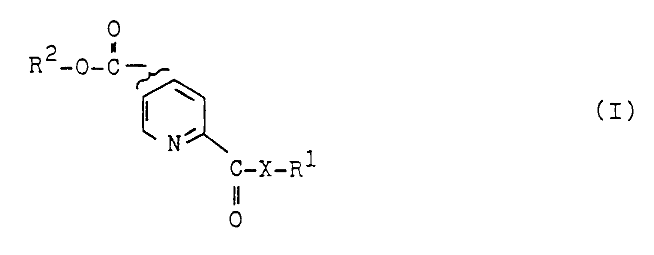 worin
R¹ verzweigtes oder unverzweigtes C₁-C₁₂-Alkyl, welches einfach oder in Falle der C₂-C₁₂-Alkyle auch mehrfach substituiert ist mit
Halogen, Hydroxy, Cyano, Carboxyl, Alkoxy, Alkoxycarbonyl, Alkylcarbonyloxy, Alkyl- oder Dialkylamino, wobei die Alkylreste 1-4 C-Atome aufweisen, die im Falle der C₃- und C₄-Alkylreste auch verzweigt sein können, Phenyl, welches seinerseits gegebenenfalls mit Halogen, Nitro, C₁-C₄-Alkyl oder C₁-C₄-Alkoxy, 1-, 2-, oder 3-fach substituiert ist, wobei bei Mehrfachsubstitutionen die Substituenten auch voneinander unabhängig verschieden sein können und wobei im Falle der C₃- und C₄-Alkyle diese auch verzweigt sein können,
oder R¹ gesättigtes C₅-C₇-Cycloalkyl, welches gegebenenfalls benzoanneliert ist,
oder R¹ Phenyl, Naphthyl oder einen 5- oder 6-gliedrigen aromatischen Ring mit 1, 2 oder 3 Stickstoff- und/oder Sauerstoff- und/oder Schwefelatomen, welche gegebenenfalls ihrerseits mit Halogen, C₁-C₄-Alkyl oder C₁-C₄-Alkoxy 1-, 2-, oder 3-fach substituiert sind, wobei bei Mehrfachsubstitutionen die Substituenten auch voneinander unabhängig verschieden sein können und wobei im Falle der C₃- und C₄-Alkyle diese auch verzweigt sein können
oder R¹ 2-Oxo-1,3-dioxolylmethyl, welches gegebenenfalls noch Methylsubstituiert ist
oder R¹ wenn X Stickstoff ist, Wasserstoff
und
R² unabhängig von R¹ Wasserstoff oder R¹ ist, wobei R² auch identisch mit R¹ sein kann
und
X Sauerstoff oder mit R³ substituierter Stickstoff, wobei R³ Wasserstoff oder C₁-C₆-Alkyl ist und gegebenenfalls zusammen mit R¹ einen heterocyclischen, gesättigten 5-, 6- oder 7-Ring bildet, wobei der heterocyclische Ring auch ein zweites Stickstoffatom beinhalten kann und wobei der heterocyclische Ring seinerseits mit Phenyl oder Phenyl-C₁-C₃-Alkyl substituiert sein kann,
bedeutet und deren physiologisch verträgliche Salze zur Anwendung als Arzneimittel.

2. Pyridin-2,4 und -2,5-dicarbonsäure-Derivate gemäß Formel I nach Anspruch 1, worin
R¹ verzweigtes oder unverzweigtes C₁-C₄-Alkyl welches einfach oder im Falle der C₃- und C₄-Alkyle auch mehrfach substituiert ist, mit C₁-C₃-Alkoxy und/oder C₁-C₃-Alkoxycarbonyl, wobei die C₃-AlkylReste auch verzweigt sein können und/oder Phenyl
oder R¹ C₅- oder C₆-Cycloalkyl, das gegebenenfalls benzoanneliert ist,
oder R¹ Phenyl, das gegebenenfalls 1-, 2- oder 3-fach Nitrosubstituiert ist,
oder R¹ 2-, 3- oder 4-Pyridyl, Naphthyl, 2- oder 3-Thienyl, Pyrazolyl, Imidazolyl oder Thiazolyl
oder R¹ 5-Methyl-2-Oxo-1,3-dioxol-4-yl-methyl bedeutet und
R² unabhängig von R¹ Wasserstoff oder R¹ ist, wobei R² auch identisch mit R¹ sein kann
und
X Sauerstoff oder mit R³ substituierter Stickstoff, wobei R³ Wasserstoff oder C₁-C₃-Alkyl ist und gegebenenfalls zusammen mit R¹ einen heterocyclischen gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
bedeutet, sowie deren physiologisch verträgliche Salze, zur Anwendung als Arzneimittel.

3. Pyridin-2,4- und -2,5-dicarbonsäure-Derivate der Formel I', 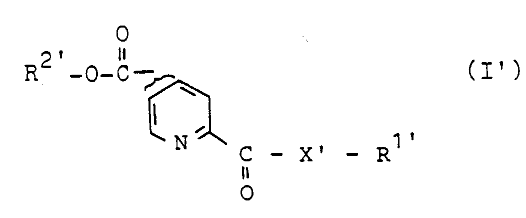 worin
R^{1'} verzweigtes oder unverzweigtes C₁-C₁₂-Alkyl, welches einfach oder im Falle von C₂-C₁₂-Alkyl auch mehrfach substituiert ist mit Halogen, Hydroxy, Cyano, Carboxyl, Alkoxy, Alkoxycarbonyl, Alkylcarbonyloxy, Alkyl- oder Dialkylamino, wobei die Alkylreste 1-4 C-Atome aufweisen, und wobei im Falle der C₃- und C₄Alkyle diese auch verzweigt sein können Phenyl, welches seinerseits gegebenenfalls mit Halogen C₁-C₄-Alkoxy oder C₁-C₄-Alkyl 1-, 2- oder 3-fach substituiert ist, wobei die genannten C₃- und C₄-Alkylreste auch verzweigt sein können und wobei bei Mehrfachsubstitutionen die Substituenten auch voneinander unabhängig verschieden sein können
oder R^{1'} gesättigtes C₅-C₇-Cycloalkyl, welches gegebenenfalls benzoanneliert ist
oder R^{1'} Phenyl, Naphthyl oder einen 5- oder 6-gliedrigen aromatischen Ring mit 1, 2 oder 3 Stickstoff- und/oder Sauerstoff- und/oder Schwefelatomen, welches gegebenenfalls seinerseits mit Halogen, Nitro, C₁-C₄-Alkyl oder C₁-C₄-Alkoxy 1-, 2- oder 3-fach substituiert ist, wobei bei Mehrfachsubstitutionen die Substituenten auch voneinander unabhängig verschieden sein können und wobei im Falle der C₃- und C₄-Alkyle diese auch verzweigt sein können,
oder R^{1'} 2-Oxo-1,3-dioxolylmethyl, welches gegebenenfalls Methyl-substituiert ist,
oder R^{1'} wenn X' Stickstoff ist, Wasserstoff,
und
R^{2'} unabhängig von R^{1'} Wasserstoff oder R^{1'} ist, wobei R^{2'} auch identisch mit R^{1'} sein kann
und
X' Sauerstoff oder mit R^{3'} substituierter Stickstoff, wobei R^{3'} Wasserstoff oder C₁-C₆-Alkyl ist und gegebenenfalls zusammen mit R^{1'} einen heterocyclischen, gesättigten 5-, 6- oder 7-Ring bildet, wobei der heterocyclische Ring auch ein zweites Stickstoffatom beinhalten kann und wobei der heterocyclische Ring seinerseits mit Phenyl oder Phenyl-C₁-C₃-alkyl substituiert sein kann,
bedeutet,
sowie deren physiologisch verträgliche Salze, ausgenommen die Verbindungen, in denen X' Sauerstoff bedeutet und R^{1'} und R^{2'} mit Chlor, Hydroxy oder Phenyl substituiertes Methyl oder Ethyl ist und Pyridin-2,5-dicarbonsäure-di(4-methoxyphenyl)ester.

4. Pyridin-2,4- und -2,5-dicarbonsäure-Derivate gemäß Formel I' nach Anspruch 3, worin
R^{1'} verzweigtes oder unverzweigtes C₁-C₄-Alkyl, welches einfach oder im Falle der C₂-C₄-Alkyle auch mehrfach substituiert ist mit Alkoxy, Alkoxycarbonyl, wobei die Alkylreste 1-3 C-Atome aufweisen und im Falle der C₃-Alkylverbindungen auch verzweigt sein können oder Phenyl
oder R^{1'} Cyclopentyl oder Cyclohexyl, die gegebenenfalls benzoanneliert sind
oder R^{1'} Phenyl, welches gegebenenfalls mit 1, 2 oder 3 Nitrogruppen substituiert ist oder Naphthyl
oder R^{1'} 2-, 3- oder 4-Pyridyl, 3-Thienyl, Pyrazolyl, Imidazolyl, Thiazolyl,
oder R^{1'} 5-Methyl-2-oxo-1,3-dioxol-4-yl-methyl
und
R^{2'} unabhängig von R^{1'}, Wasserstoff oder R^{1'} ist, wobei R^{2'} auch identisch mit R^{1'} sein kann
und
X' Sauerstoff oder mit R^{3'} substituierter Stickstoff, wobei R^{3'} Wasserstoff oder C₁-C₃-Alkyl ist und gegebenenfalls zusammen mit R^{1'} einen heterocyclischen, gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
bedeutet,
sowie deren physiologisch verträgliche Salze, ausgenommen die Verbindungen, in denen X' Sauerstoff bedeutet und R^{1'} und R^{2'} mit Chlor, Hydroxy oder Phenyl substituiertes Methyl oder Ethyl ist und Pyridin-2,5-dicarbonsäure-di(4-methoxyphenyl)ester.

5. Verfahren zur Herstellung von Verbindungen der Formel I' nach Anspruch 3, dadurch gekennzeichnet, daß man
a) eine Verbindung der Formel II 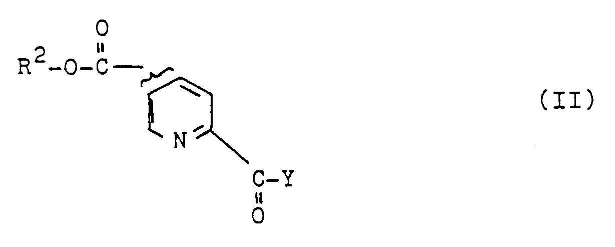 mit einer Verbindung der Formel III
HX'-R^{1'} (III
)
umsetzt, wobei R^{1'}, R^{2'} und X' die zu Formel I' angegebenen Bedeutungen haben und Y Halogen oder Hydroxy ist oder
b) eine Verbindung der Formel IV 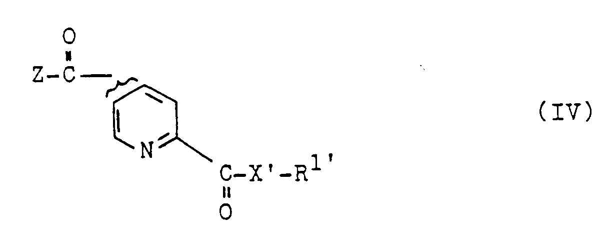 mit einer Verbindung der Formel V
HO-R^{2'} (V)
umsetzt, wobei R^{1'} , R^{2'} und X' die zu Formel I' angegebenen Bedeutungen haben und Z Halogen ist oder
c) eine Verbindung der Formel VI 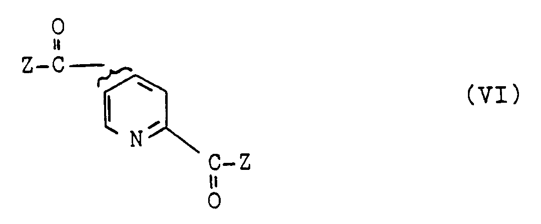 mit einem Alkohol HO-R^{2'} oder einem Alkohol der Formel VII
HO-R^{1'} (VII)
umsetzt, wobei R^{1'} und R^{2'} die zu Formel I' angegebenen Bedeutungen haben und Z Halogen ist oder
d) ein Alkalisalz der Pyridin-2,4- oder -2,5-dicarbonsäure mit einem Halogenid der Formel VIII
R^{1'}-Z (VIII)
unsetzt, wobei R^{1'} die zu Formel I' angegebenen Bedeutungen hat und Z Halogen ist
und die Reaktionsprodukte gegebenenfalls in ihre physiologisch verträglichen Salze überführt.

6. Verfahren nach Anspruch 5, dadurch gekennzeichnet, daß in Verfahrensvariante a) die Umsetzung unter gleichzeitiger Hinzugabe von Dialkylcarbodiimid erfolgt, wobei die Dialkylreste 1 bis 8 C-Atome aufweisen, die im Falle der C₃-C₈-Verbindungen auch verzweigt oder cyclisch sein können.

7. Verbindungen nach einem der Ansprüche 1 bis 4 zur Inhibierung der Prolin- und Lysinhydroxylase.

8. Verbindungen nach Anspruch 1 bis 4 zur Anwendung als Fibrosuppressiva und Immunsuppressiva.

9. Arzneimittel, enthaltend eine Verbindung der Formel I oder I' gemäß Anspruch 1 oder 3 mit verträglichen pharmazeutischen Trägern.

10. Verfahren zur Herstellung von Arzneimitteln zur Beeinflussung des Stoffwechsels von Collagen und collagenähnlichen Stoffen bzw. der Biosynthese von C1_{q}, dadurch gekennzeichnet, daß man in das Arzneimittel eine Verbindung der Formel I oder I' gemäß Anspruch 1 oder 3 einverleibt.

11. Pyridin-2,4- und -2,5-dicarbonsäure-Derivate der Formel I' gemäß Anspruch 3 worin R^{1'} verzweigtes oder unverzweigtes C₁-C₄-Alkyl, welches substituiert ist mit Alkoxycarbonyl, wobei die Alkylreste 1 - 3 C-Atome aufweisen, die im Falle der C₃-Alkylreste auch verzweigt sein können, und R^{2'} unabhängig von R^{1'} Wasserstoff oder R^{1'} ist, wobei R^{2'} auch identisch mit R^{1'} sein kann und
X' Sauerstoff bedeutet,
sowie deren physiologisch verträgliche Salze.

12. Pyridin-2,4- und -2,5-dicarbonsäure-Derivate der Formel I' gemäß Anspruch 3, worin R^{1'} und R^{2'} 1-Isopropoxycarbonlethyl-Gruppen sind und X' Sauerstoff bedeutet, sowie deren physiologisch verträgliche Salze.

## Patentansprüche (Patentansprüche für folgende(n) Vertragsstaat(en): ES, GR)

1. Verfahren zur Herstellung von Arzneimitteln, dadurch gekennzeichnet, daß man Pyridin-2,4- und -2,5-dicarbonsäure-Derivate der Formel I 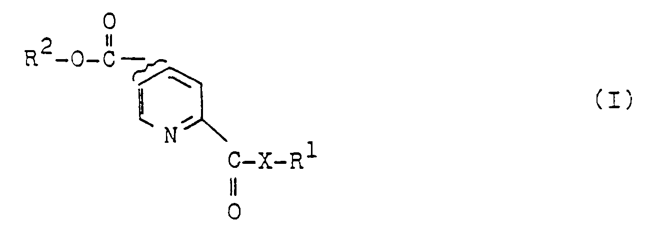 worin
R¹ verzweigtes oder unverzweigtes C₁-C₁₂-Alkyl, welches einfach oder im Falle der C₂-C₁₂-Alkyle auch mehrfach substituiert ist mit
Halogen, Hydroxy, Cyano, Carboxyl, Alkoxy, Alkoxycarbonyl, Alkylcarbonyloxy, Alkyl- oder Dialkylamino, wobei die Alkylreste 1-4 C-Atome aufweisen, die im Falle der C₃- und C₄-Alkylreste auch verzweigt sein können, Phenyl, welches seinerseits gegebenenfalls mit Halogen, Nitro, C₁-C₄-Alkyl oder C₁-C₄-Alkoxy, 1-, 2-, oder 3-fach substituiert ist, wobei bei Mehrfachsubstitutionen die Substituenten auch voneinander unabhängig verschieden sein können und wobei im Falle der C₃- und C₄-Alkyle diese auch verzweigt sein können,
oder R¹ gesättigtes C₅-C₇-Cycloalkyl, welches gegebenenfalls benzoanneliert ist,
oder R¹ Phenyl, Naphthyl oder einen 5- oder 6-gliedrigen aromatischen Ring mit 1,2 oder 3 Stickstoff- und/oder Sauerstoff- und/oder Schwefelatomen, welche gegebenenfalls ihrerseits mit Halogen, C₁-C₄-Alkyl oder C₁-C₄-Alkoxy 1-, 2- oder 3-fach substituiert sind,
wobei bei Mehrfachsubstitutionen die Substituenten auch voneinander unabhängig verschieden sein können und wobei im Falle der C₃- und C₄-Alkyle diese auch verzweigt sein können
oder R¹ 2-Oxo-1,3-dioxolylmethyl, welches gegebenenfalls noch Methylsubstituiert ist
oder R¹ wenn X Stickstoff ist, Wasserstoff
und
R² unabhängig von R¹ Wasserstoff oder R¹ ist, wobei R² auch identisch mit R¹ sein kann
und
X Sauerstoff oder mit R³ substituierter Stickstoff, wobei R³ Wasserstoff oder C₁-C₆-Alkyl ist und gegebenenfalls zusammen mit R¹ einen heterocyclischen, gesättigten 5-, 6- oder 7-Ring bildet, wobei der heterocyclische Ring auch ein zweites Stickstoffatom beinhalten kann und wobei der heterocyclische Ring seinerseits mit Phenyl oder Phenyl-C₁-C₃-Alkyl substituiert sein kann,
bedeutet und deren physiologisch verträgliche Salze in eine geeignete Darreichungsform bringt.

2. Verfahren zur Herstellung von Arzneimitteln, dadurch gekennzeichnet, daß man Pyridin-2,4- und -2,5-dicarbonsäure-Derivate gemäß Formel I nach Anspruch 1, worin
R¹ verzweigtes oder unverzweigtes C₁-C₄-Alkyl welches einfach oder im Falle der C₃- und C₄-Alkyle auch mehrfach substituiert ist, mit C₁-C₃-Alkoxy und/oder C₁-C₃-Alkoxycarbonyl, wobei die C₃-AlkylReste auch verzweigt sein können und/oder Phenyl
oder R¹ C₅- oder C₆-Cycloalkyl, das gegebenenfalls benzoanneliert ist,
oder R¹ Phenyl, das gegebenenfalls 1-, 2- oder 3-fach Nitrosubstituiert ist,
oder R¹ 2-, 3- oder 4-Pyridyl, Naphthyl, 2- oder 3-Thienyl, Pyrazolyl, Imidazolyl oder Thiazolyl
oder R¹ 5-Methyl-2-Oxo-1,3-dioxol-4-yl-methyl bedeutet und
R² unabhängig von R¹ Wasserstoff oder R¹ ist, wobei R² auch identisch mit R¹ sein kann
und
X Sauerstoff oder mit R³ substituierter Stickstoff, wobei R³ Wasserstoff oder C₁-C₃-Alkyl ist und gegebenenfalls zusammen mit R¹ einen heterocyclischen gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
bedeutet, sowie deren physiologisch verträgliche Salze, in eine geeignete Darreichungsform bringt.

3. Verfahren zur Herstellung von Pyridin-2,4- und -2,5-dicarbonsäure-Derivaten der Formel I', 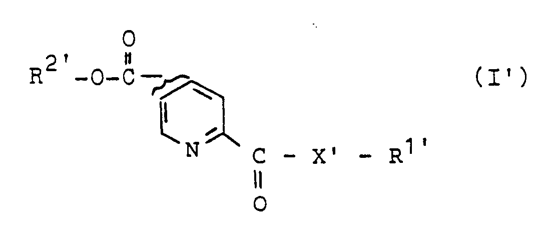 worin
R^{1'} verzweigtes oder unverzweigtes C₁-C₁₂-Alkyl, welches einfach oder in Falle von C₂-C₁₂-Alkyl auch mehrfach substituiert ist mit Halogen, Hydroxy, Cyano, Carboxyl, Alkoxy, Alkoxycarbonyl, Alkylcarbonyloxy, Alkyl- oder Dialkylamino, wobei die Alkylreste 1-4 C-Atome aufweisen, und wobei im Falle der C₃- und C₄Alkyle diese auch verzweigt sein können Phenyl, welches seinerseits gegebenenfalls mit Halogen C₁-C₄-Alkoxy oder C₁-C₄-Alkyl 1-, 2- oder 3-fach substituiert ist, wobei die genannten C₃- und C₄-Alkylreste auch verzweigt sein können und wobei bei Mehrfachsubstitutionen die Substituenten auch voneinander unabhängig verschieden sein können
oder R^{1'} gesättigtes C₅-C₇-Cycloalkyl, welches gegebenenfalls benzoanneliert ist
oder R^{1'} Phenyl, Naphthyl oder einen 5- oder 6-gliedrigen aromatischen Ring mit 1, 2 oder 3 Stickstoff- und/oder Sauerstoff- und/oder Schwefelatomen, welches gegebenenfalls seinerseits mit Halogen, Nitro, C₁-C₄-Alkyl oder C₁-C₄-Alkoxy 1-, 2- oder 3-fach substituiert ist, wobei bei Mehrfachsubstitutionen die Substituenten auch voneinander unabhängig verschieden sein können und wobei im Falle der C₃- und C₄-Alkyle diese auch verzweigt sein können,
oder R^{1'} 2-Oxo-1,3-dioxolylmethyl, welches gegebenenfalls Methyl-substituiert ist,
oder R^{1'} wenn X' Stickstoff ist, Wasserstoff,
und
R^{2'} unabhängig von R^{1'} Wasserstoff oder R^{1'} ist, wobei R^{2'} auch identisch mit R^{1'} sein kann
und
X' Sauerstoff oder mit R^{3'} substituierter Stickstoff, wobei R^{3'} Wasserstoff oder C₁-C₆-Alkyl ist und gegebenenfalls zusammen mit R^{1'} einen heterocyclischen, gesättigten 5-, 6- oder 7-Ring bildet, wobei der heterocyclische Ring auch ein zweites Stickstoffatom beinhalten kann und wobei der heterocyclische Ring seinerseits mit Phenyl oder Phenyl-C₁-C₃-alkyl substituiert sein kann,
bedeutet,
sowie deren physiologisch verträglichen Salzen, ausgenommen die Verbindungen, in denen X' Sauerstoff bedeutet und R^{1'} und R^{2'} mit Chlor, Hydroxy oder Phenyl substituiertes Methyl oder Ethyl ist und Pyridin-2,5-dicarbonsäure-di(4-methoxyphenyl)ester, dadurch gekennzeichnet, daß man
a) eine Verbindung der Formel II 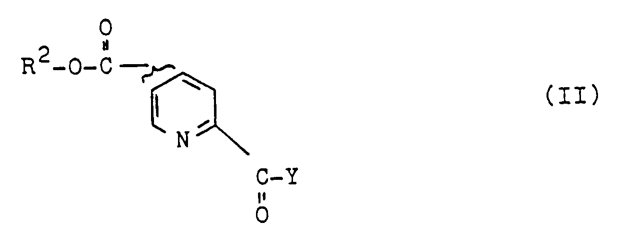 mit einer Verbindung der Formel III
HX'-R^{1'} (III)
unsetzt, wobei R^{1'}, R^{2'} und X' die zu Formel I' angegebenen Bedeutungen haben und Y Halogen oder Hydroxy ist oder
b) eine Verbindung der Formel IV 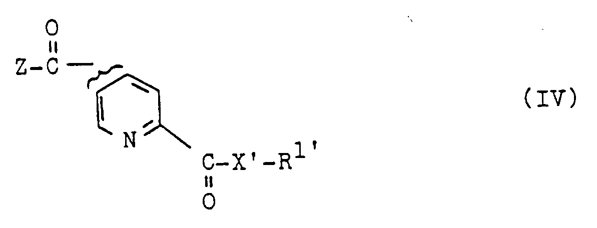 mit einer Verbindung der Formel V
HO-R^{2'} (V)
umsetzt, wobei R^{1'}, R^{2'} und X' die zu Formel I' angegebenen Bedeutungen haben und Z Halogen ist oder
c) eine Verbindung der Formel VI 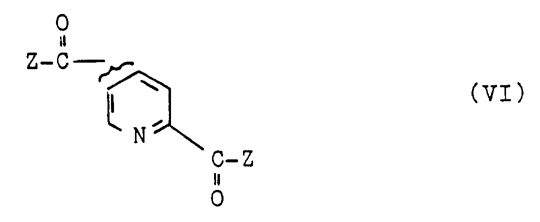 mit einem Alkohol HO-R^{2'} oder einem Alkohol der Formel VII
HO-R^{1'} (VII)
umsetzt, wobei R^{1'} und R^{2'} die zu Formel I' angegebenen Bedeutungen haben und Z Halogen ist oder
d) ein Alkalisalz der Pyridin-2,4- oder -2,5-dicarbonsäure mit einem Halogenid der Formel VIII
R^{1'}-Z (VIII)
umsetzt, wobei R^{1'} die zu Formel I' angegebenen Bedeutungen hat und Z Halogen ist
und die Reaktionsprodukte gegebenenfalls in ihre physiologisch verträglichen Salze überführt.

4. Verfahren nach Anspruch 3 zur Herstellung von Pyridin-2,4- und -2,5-dicarbonsäure-Derivaten gemäß Formel I' worin
R^{1'} verzweigtes oder unverzweigtes C₁-C₄-Alkyl, welches einfach oder im Falle der C₂-C₄-Alkyle auch mehrfach substituiert ist mit Alkoxy, Alkoxycarbonyl, wobei die Alkylreste 1-3 C-Atome aufweisen und im Falle der C₃-Alkylverbindungen auch verzweigt sein können oder Phenyl
oder R^{1'} Cyclopentyl oder Cyclohexyl, die gegebenenfalls benzoanneliert sind
oder R^{1'} Phenyl, welches gegebenenfalls mit 1, 2 oder 3 Nitrogruppen substituiert ist oder Naphthyl
oder R^{1'} 2-, 3- oder 4-Pyridyl, 3-Thienyl, Pyrazolyl, Imidazolyl, Thiazolyl,
oder R^{1'} 5-Methyl-2-oxo-1,3-dioxol-4-yl-methyl
und
R^{2'} unabhängig von R^{1'}, Wasserstoff oder R^{1'} ist, wobei R^{2'} auch identisch mit R^{1'} sein kann
und
X' Sauerstoff oder mit R^{3'} substituierter Stickstoff, wobei R^{3'} Wasserstoff oder C₁-C₃-Alkyl ist und gegebenenfalls zusammen mit R^{1'} einen heterocyclischen, gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
bedeutet,
sowie deren physiologisch verträglichen Salzen, ausgenommen die Verbindungen, in denen X' Sauerstoff bedeutet und R^{1'} und R^{2'} mit Chlor, Hydroxy oder Phenyl substituiertes Methyl oder Ethyl ist und Pyridin-2,5-dicarbonsäure-di(4-methoxyphenyl)ester.

5. Verfahren nach Anspruch 3 oder 4 dadurch gekennzeichnet, daß in Verfahrensvariante a) die Umsetzung unter gleichzeitiger Hinzugabe von Dialkylcarbodiimid erfolgt, wobei die Dialkylreste 1 bis 8 C-Atome aufweisen, die im Falle der C₃-C₈-Verbindungen auch verzweigt oder cyclisch sein können.

6. Verfahren zur Herstellung von Arzneimitteln zur Inhibierung der Prolin- und Lysinhydroxylase, dadurch gekennzeichnet, daß man Verbindungen der Formeln I oder I' gemäß Anspruch 1 oder 3 in eine geeignete Darreichungsform bringt.

7. Verfahren zur Herstellung von Arzneimitteln zur Anwendung als Fibrosuppressiva und Immunosuppressiva, dadurch gekennzeichnet, daß man Verbindungen der Formel I oder I' gemäß Anspruch 1 oder 3 in eine geeignete Darreichungsform bringt.

8. Verfahren zur Herstellung von Arzneimitteln zur Beeinflussung des Stoffwechsels von Collagen und collagenähnlichen Stoffen bzw. der Biosynthese von C1_{q}, dadurch gekennzeichnet, daß man eine Verbindung der Formel I oder I' gemäß Anspruch 1 oder 3 in eine geeignete Darreichungsform bringt.

9. Verfahren nach Anspruch 3 zur Herstellung von Pyridin-2,4- und -2,5-dicarbonsäure-Derivatender Formel I' gemäß Anspruch 3 worin R^{1'} verzweigtes oder unverzweigtes C₁-C₄-Alkyl, welches substituiert ist mit Alkoxycarbonyl, wobei die Alkylreste 1 - 3 C-Atome aufweisen und R^{2'} unabhängig von R^{1'} Wasserstoff oder R^{1'} ist, wobei R^{2'} auch identisch mit R^{1'} sein kann und
X' Sauerstoff bedeutet,
sowie deren physiologisch verträglichen Salzen.

10. Verfahren nach Anspruch 3 zur Herstellung von Pyridin-2,4- und -2,5-dicarbonsäure-Derivatender Formel I', gemäß Anspruch 3 worin R^{1'} und R^{2'} 1-Isopropoxycarbonylethyl-Gruppen sind und X' Sauerstoff bedeutet, sowie deren physiologisch vertäglichen Salzen.

## Claims (Claims for the following Contracting State(s): AT, BE, CH, DE, FR, GB, IT, LI, LU, NL, SE)

1. A pyridine-2,4- or -2,5-dicarboxylic acid derivative of the formula I 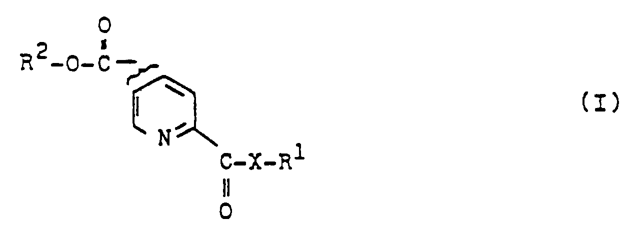 in which
R¹ denotes branched or unbranched C₁-C₁₂-alkyl which is monosubstituted or else, in the case of the C₂-C₁₂-alkyl radicals, polysubstituted by halogen, hydroxyl, cyano, carboxyl, alkoxy, alkoxycarbonyl, alkylcarbonyloxy or alkyl- or dialkylamino, the alkyl radicals containing 1-4 carbon atoms and, in the case of the C₃- and C₄-alkyl radicals, it also being possible for them to be branched, phenyl, which is in turn optionally mono-, di- or trisubstituted by halogen, nitro, C₁-C₄-alkyl or C₁-C₄-alkoxy, it also being possible, in the case of polysubstitution, for the substituents to differ independently of one another and it also being possible, in the case of the C₃- and C₄-alkyl radicals, for these to be branched,
or R¹ denotes saturated C₅-C₇-cycloalkyl, which is optionally benzo-fused,
or R¹ denotes phenyl, naphthyl or a 5- or 6-membered aromatic ring having 1, 2 or 3 nitrogen and/or oxygen and/or sulfur atoms, which in turn are optionally mono-, di- or trisubstituted by halogen, C₁-C₄-alkyl or C₁-C₄-alkoxy, it also being possible, in the case of polysubstitution, for the substituents to differ independently of one another and it also being possible, in the case of the C₃-and C₄-alkyl radicals, for these to be branched,
or R¹ denotes 2-oxo-1,3-dioxolylmethyl, which is optionally also methyl-substituted,
or R¹,if X is nitrogen, denotes hydrogen,
and
R² independently of R¹ is hydrogen or R¹, it also being possible for R² to be identical to R¹,
and
X denotes oxygen or R³-substituted nitrogen, in which R³ is hydrogen or C₁-C₆-alkyl or optionally together with R¹ forms a heterocyclic saturated 5-, 6- or 7-membered ring, it also being possible for the heterocyclic ring to include a second nitrogen atom and it being possible for the heterocyclic ring in turn to be substituted by phenyl or phenyl-C₁-C₃-alkyl,
or a physiologically tolerated salt thereof for use as a pharmaceutical.

2. A pyridine-2,4- or -2,5-dicarboxylic acid derivative of formula I as claimed in claim 1, in which
R¹ denotes branched or unbranched C₁-C₄-alkyl which is monosubstituted or else, in the case of the C₃- and C₄-alkyl radicals, polysubstituted by C₁-C₃-alkoxy and/or C₁-C₃-alkoxycarbonyl, it also being possible for the C₃-alkyl radicals to be branched, and/or phenyl,
or R¹ denotes C₅- or C₆-cycloalkyl, which is optionally benzo-fused,
or R¹ denotes phenyl, which is optionally mono-, di- or trisubstituted by nitro,
or R¹ denotes 2-, 3- or 4-pyridyl, naphthyl, 2-or 3-thienyl, pyrazolyl, imidazolyl or thiazolyl,
or R¹ denotes 5-methyl-2-oxo-1,3-dioxol-4-yl-methyl
and
R² independently of R¹ is hydrogen or R¹, it also being possible for R² to be identical to R¹,
and
X denotes oxygen or R³-substituted nitrogen, in which R³ is hydrogen or C₁-C₃-alkyl or optionally together with R¹, forms a heterocyclic saturated 6-membered ring, it also being possible for the heterocyclic 6-membered ring to include a second nitrogen atom and to be substituted in turn by phenyl or phenyl-C₁-C₃-alkyl,
or a physiologically tolerated salt thereof, for use as a pharmaceutical.

3. A pyridine-2,4- or -2,5-dicarboxylic acid derivative of the formula I' 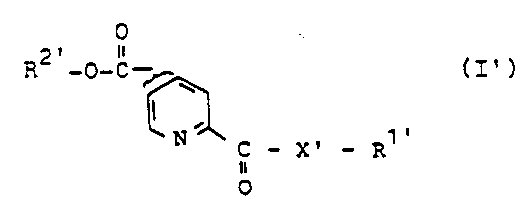 in which
R^{1'} denotes branched or unbranched C₁-C₁₂-alkyl which is monosubstituted or else, in the case of C₂-C₁₂-alkyl, polysubstituted by halogen, hydroxyl, cyano, carboxyl, alkoxy, alkoxycarbonyl, alkylcarbonyloxy or alkyl- or dialkylamino, the alkyl radicals containing 1-4 carbon atoms and it also being possible, in the case of the C₃- and C₄-alkyl radicals, for these to be branched, phenyl, which is in turn optionally mono-, di- or trisubstituted by halogen, C₁-C₄-alkoxy or C₁-C₄-alkyl, it also being possible for the C₃- and C₄-alkyl radicals mentioned to be branched and it also being possible, in the case of polysubstitution, for the substituents to differ independently of one another,
or R^{1'}denotes saturated C₅-C₇-cycloalkyl, which is optionally benzo-fused,
or R^{1'} denotes phenyl, naphthyl or a 5- or 6-membered aromatic ring having 1, 2 or 3 nitrogen and/or oxygen and/or sulfur atoms, which is in turn optionally mono-, di- or trisubstituted by halogen, nitro, C₁-C₄-alkyl or C₁-C₄-alkoxy, it also being possible, in the case of polysubstitution, for the substituents to differ independently of one another and it also being possible, in the case of the C₃- and C₄-alkyl radicals, for these to be branched,
or R^{1'} denotes 2-oxo-1,3-dioxolylmethyl, which is optionally methyl-substituted,
or R^{1'}, if X' is nitrogen, denotes hydrogen,
and
R^{2'} independently of R^{1'} is hydrogen or R^{1'}, it also being possible for R^{2'} to be identical to R^{1'},
and
X' denotes oxygen or R^{3'}-substituted nitrogen, in which R^{3'} is hydrogen or C₁-C₆-alkyl or, optionally together with R^{1'}, forms a heterocyclic, saturated 5-, 6- or 7-membered ring, it also being possible for the heterocyclic ring to include a second nitrogen atom and it being possible for the heterocyclic ring in turn to be substituted by phenyl or phenyl-C₁-C₃-alkyl,
or a physiologically tolerated salt thereof, excluding the compounds in which X' denotes oxygen and R^{1'} and R^{2'} are methyl or ethyl which is substituted by chlorine, hydroxyl or phenyl, and di(4-methoxyphenyl) pyridine-2,5-dicarboxylate.

4. A pyridine-2,4- or -2,5-dicarboxylic acid derivative of formula I' as claimed in claim 3, in which
R^{1'} denotes branched or unbranched C₁-C₄-alkyl, which is monosubstituted or else, in the case of the C₂-C₄-alkyl radicals, polysubstituted by alkoxy or alkoxycarbonyl, the alkyl radicals containing 1-3 carbon atoms and it also being possible, in the case of the C₃-alkyl compounds, for these to be branched, or phenyl
or R^{1'} denotes cyclopentyl or cyclohexyl, which are optionally benzo-fused,
or R^{1'} denotes phenyl, which is optionally substituted by 1, 2 or 3 nitro groups, or naphthyl,
or R^{1'} denotes 2-, 3- or 4-pyridyl, 3-thienyl, pyrazolyl, imidazolyl or thiazolyl,
or R^{1'} denotes 5-methyl-2-oxo-1,3-dioxol-4-yl-methyl
and
R^{2'} independently of R^{1'} is hydrogen or R^{1'}, it also being possible for R^{2'} to be identical to R^{1'},
and
X' denotes oxygen or R^{3'}-substituted nitrogen, in which R^{3'} is hydrogen or C₁-C₃-alkyl or optionally together with R^{1'} forms a heterocyclic, saturated 6-membered ring, it also being possible for the heterocyclic 6-membered ring to include a second nitrogen atom and in turn to be substituted by phenyl or phenyl-C₁-C₃-alkyl,
or a physiologically tolerated salt thereof, excluding the compounds in which X' denotes oxygen and R^{1'} and R^{2'} are methyl or ethyl which is substituted by chlorine, hydroxyl or phenyl, and di-(4methoxyphenyl) pyridine-2,5-dicarboxylate.

5. A process for the preparation of a compound of the formula I' as claimed in claim 3, which comprises
a) reacting a compound of the formula II 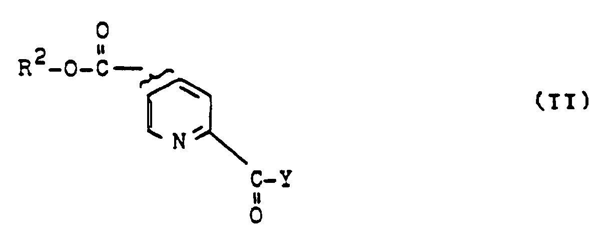 with a compound of the formula III
HX'-R^{1'} (III)
in which R^{1'}, R^{2'} and X' have the meanings given in the case of formula I' and Y is halogen or hydroxyl, or
b) reacting a compound of the formula IV 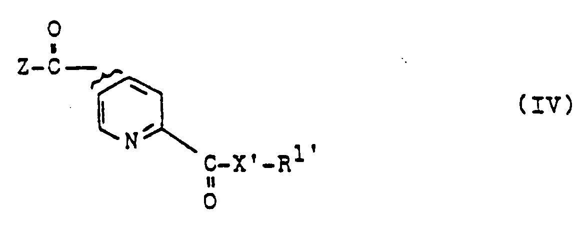 with a compound of the formula V
HO-R^{2'} (V)
in which R^{1'}, R^{2'} and X' have the meanings given in the case of formula I' and Z is halogen, or
c) reacting a compound of the formula VI 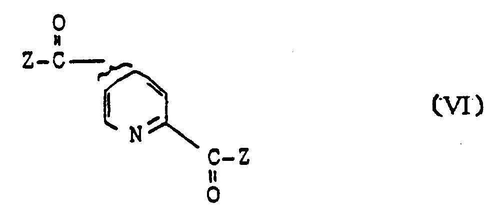 with an alcohol HO-R^{2'} or an alcohol of the formula VII
HO-R^{1'} (VII)
in which R^{1'} and R^{2'} have the meanings given in the case of formula I' and Z is halogen, or
d) reacting an alkali metal salt of pyridine-2,4- or -2,5-dicarboxylic acid with a halide of the formula VIII
R^{1'}-Z (VIII)
in which R^{1'} has the meanings given in the case of formula I' and Z is halogen,
and, if appropriate, converting the reaction product into one of its physiologically tolerated salts.

6. The process as claimed in claim 5, wherein, in process variant a), the reaction is carried out with simultaneous addition of a dialkylcarbodiimide, the dialkyl radicals containing 1 to 8 carbon atoms and it also being possible, in the case of the C₃-C₈ compounds for these to be branched or cyclic.

7. A compound as claimed in one of claims 1 to 4 for inhibiting proline hydroxylase and lysine hydroxylase.

8. A compound as claimed in claim 1 to 4 for use as a fibrosuppressant and immunosuppressant.

9. A pharmaceutical containing a compound of the formula I or I' as claimed in claim 1 or 3 with tolerated pharmaceutical excipients.

10. A process for the preparation of a pharmaceutical for influencing the metabolism of collagen and collagen-like substances or the biosynthesis of C1_{q}, which comprises incorporating a compound of the formula I or I' as claimed in claim 1 or 3 into the pharmaceutical.

11. A pyridine-2,4- or -2,5-dicarboxylic acid derivative of the formula I' as claimed in claim 3, in which R^{1'} is branched or unbranched C₁-C₄-alkyl, which is substituted by alkoxycarbonyl, the alkyl radicals containing 1 - 3 carbon atoms and it also being possible, in the case of the C₃-alkyl radicals, for these to be branched, and R^{2'} independently of R^{1'} is hydrogen or R^{1'}, it also being possible for R^{2'} to be identical to R^{1'}, and X' denotes oxygen, or a physiologically tolerated salt thereof.

12. A pyridine-2,4- or -2,5-dicarboxylic acid derivative of the formula I' as claimed in claim 3, in which R^{1'} and R^{2'} are 1-isopropoxycarbonylethyl groups and X' denotes oxygen, or a physiologically tolerated salt thereof.

## Claims (Claims for the following Contracting State(s): ES, GR)

1. A process for the preparation of a pharmaceutical, which comprises bringing a pyridine-2,4- or -2,5-dicarboxylic acid derivative of the formula I 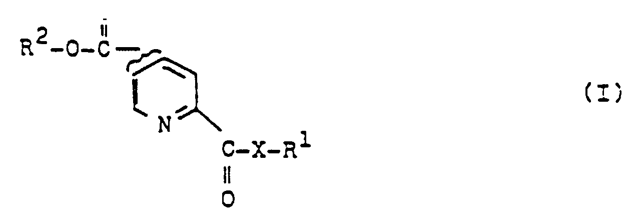 in which
R¹ denotes branched or unbranched C₁-C₁₂-alkyl which is monosubstituted or else, in the case of the C₂-C₁₂-alkyl radicals, polysubstituted by
halogen, hydroxyl, cyano, carboxyl, alkoxy, alkoxy-carbonyl, alkylcarbonyloxy or alkyl- or dialkylamino, the alkyl radicals containing 1-4 carbon atoms and, in the case of the C₃- and C₄-alkyl radicals, it also being possible for them to be branched, phenyl, which is in turn optionally mono-, di- or trisubstituted by halogen, nitro, C₁-C₄-alkyl or C₁-C₄-alkoxy, it also being possible, in the case of polysubstitution, for the substituents to differ independently of one another and it also being possible, in the case of the C₃- and C₄-alkyl radicals, for these to be branched,
or R¹ denotes saturated C₅-C₇-cycloalkyl, which is optionally benzo-fused,
or R¹ denotes phenyl, naphthyl or a 5- or 6-membered aromatic ring having 1, 2 or 3 nitrogen and/or oxygen and/or sulfur atoms, which in turn are optionally mono-, di- or trisubstituted by halogen, C₁-C₄-alkyl or C₁-C₄-alkoxy, it also being possible, in the case of polysubstitution, for the substituents to differ independently of one another and it also being possible, in the case of the C₃- and C₄-alkyl radicals, for these to be branched,
or R¹ denotes 2-oxo-1,3-dioxolylmethyl, which is optionally also methyl-substituted,
or R¹, if X is nitrogen, denotes hydrogen,
and
R² independently of R¹ is hydrogen or R¹, it also being possible for R² to be identical to R¹,
and
X denotes oxygen or R³-substituted nitrogen, in which R³ is hydrogen or C₁-C₆-alkyl or optionally together with R¹ forms a heterocyclic saturated 5-, 6- or 7-membered ring, it also being possible for the heterocyclic ring to include a second nitrogen atom and it being possible for the heterocyclic ring in turn to be substituted by phenyl or phenyl-C₁-C₃-alkyl,
or a physiologically tolerated salt thereof, into a suitable administration form.

2. A process for the preparation of a pharmaceutical, which comprises bringing a pyridine-2,4- or -2,5-dicarboxylic acid derivative according to formula I as in claim 1, in which
R¹ denotes branched or unbranched C₁-C₄-alkyl which is monosubstituted or else, in the case of the C₃-and C₄-alkyl radicals, polysubstituted by C₁-C₃-alkoxy and/or C₁-C₃-alkoxycarbonyl, it also being possible for the C₃-alkyl radicals to be branched, and/or phenyl,
or R¹ denotes C₅- or C₆-cycloalkyl, which is optionally benzo-fused,
or R¹ denotes phenyl, which is optionally mono-, di- or trisubstituted by nitro,
or R¹ denotes 2-, 3- or 4-pyridyl, naphthyl, 2- or 3-thienyl, pyrazolyl, imidazolyl or thiazolyl,
or R¹ denotes 5-methyl-2-oxo-1,3-dioxol-4-yl-methyl
and
R² independently of R¹ is hydrogen or R¹, it also being possible for R² to be identical to R¹,
and
X denotes oxygen or R³-substituted nitrogen, in which R³ is hydrogen or C₁-C₃-alkyl or optionally together with R¹ forms a heterocyclic saturated 6-membered ring, it also being possible for the heterocyclic 6-membered ring to include a second nitrogen atom and to be substituted in turn by phenyl or phenyl-C₁-C₃-alkyl,
or a physiologically tolerated salt thereof, into a suitable administration form.

3. A process for the preparation of a pyridine-2,4- or -2,5-dicarboxylic acid derivative of the formula I' 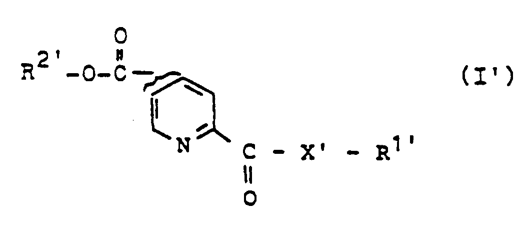 in which
R^{1'} denotes branched or unbranched C₁-C₁₂-alkyl which is monosubstituted or else, in the case of C₂-C₁₂-alkyl, polysubstituted by halogen, hydroxyl, cyano, carboxyl, alkoxy, alkoxycarbonyl, alkylcarbonyloxy or alkyl- or dialkylamino, the alkyl radicals containing 1-4 carbon atoms and it also being possible, in the case of the C₃- and C₄-alkyl radicals, for these to be branched, phenyl, which is in turn optionally mono-, di- or trisubstituted by halogen, C₁-C₄-alkoxy or C₁-C₄-alkyl, it also being possible for the C₃- and C₄-alkyl radicals mentioned to be branched and it also being possible, in the case of polysubstitution, for the substituents to differ independently of one another,
or R^{1'} denotes saturated C₅-C₇-cycloalkyl, which is optionally benzo-fused,
or R^{1'} denotes phenyl, naphthyl or a 5- or 6-membered aromatic ring having 1, 2 or 3 nitrogen and/or oxygen and/or sulfur atoms, which is in turn optionally mono-, di- or trisubstituted by halogen, nitro, C₁-C₄-alkyl or C₁-C₄-alkoxy, it also being possible, in the case of polysubstitution, for the substituents to differ independently of one another and it also being possible, in the case of the C₃- and C₄-alkyl radicals, for these to be branched,
or R^{1'} denotes 2-oxo-1,3-dioxolylmethyl, which is optionally methyl-substituted,
or R^{1'}, if X' is nitrogen, denotes hydrogen,
and
R^{2'} independently of R^{1'} is hydrogen or R^{1'}, it also being possible for R^{2'} to be identical to R^{1'},
and
X' denotes oxygen or R^{3'}-substituted nitrogen, in which R^{3'} is hydrogen or C₁-C₆-alkyl or optionally together with R^{1'} forms a heterocyclic, saturated 5-, 6- or 7-membered ring, it also being possible for the heterocyclic ring to include a second nitrogen atom and it being possible for the heterocyclic ring in turn to be substituted by phenyl or phenyl-C₁-C₃-alkyl,
or a physiologically tolerated salt thereof, excluding the compounds in which X' denotes oxygen and R^{1'} and R^{2'} are methyl or ethyl which is substituted by chlorine, hydroxyl or phenyl, and di(4-methoxyphenyl) pyridine-2,5-dicarboxylate which comprises
a) reacting a compound of the formula II 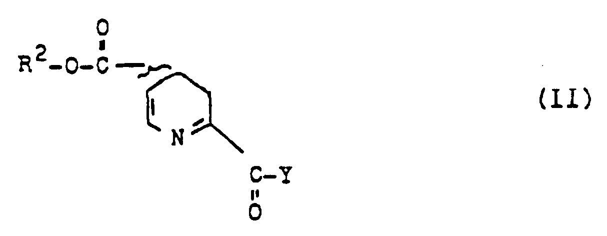 with a compound of the formula III
HX'-R^{1'} (III)
in which R^{1'}, R^{2'} and X' have the meanings given in the case of formula I' and Y is halogen or hydroxyl, or
b) reacting a compound of the formula IV 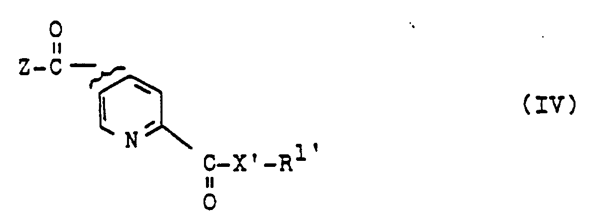 with a compound of the formula V
HO-R^{2'} (V)
in which R^{1'}, R^{2'} and X' have the meanings given in the case of formula I' and Z is halogen, or
c) reacting a compound of the formula VI 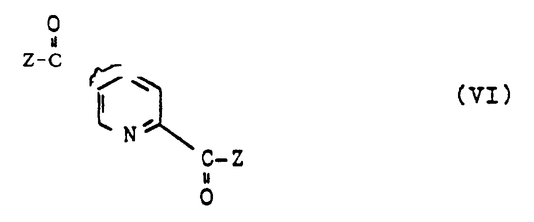 with
an alcohol HO-R^{2'} or an alcohol of the formula VII
HO-R^{1'} (VII)
in which R^{1'} and R^{2'} have the meanings given in the case of formula I' and Z is halogen, or
d) reacting an alkali metal salt of pyridine-2,4- or -2,5-dicarboxylic acid with a halide of the formula VIII
R^{1'}-Z (VIII)
in which R^{1'} has the meanings given in the case of formula I' and Z is halogen,
and, if appropriate, converting the reaction product into one of its physiologically tolerated salts.

4. The process as claimed in claim 3 for the preparation of a pyridine-2,4- or -2,5-dicarboxylic acid derivative of formula I', in which
R^{1'} denotes branched or unbranched C₁-C₄-alkyl, which is monosubstituted or else, in the case of the C₂-C₄-alkyl radicals, polysubstituted by alkoxy or alkoxycarbonyl, the alkyl radicals containing 1-3 carbon atoms and it also being possible, in the case of the C₃-alkyl compounds, for these to be branched, or phenyl
or R^{1'} denotes cyclopentyl or cyclohexyl, which are optionally benzo-fused,
or R^{1'} denotes phenyl, which is optionally substituted by 1, 2 or 3 nitro groups, or naphthyl,
or R^{1'} denotes 2-, 3- or 4-pyridyl, 3-thienyl, pyrazolyl, imidazolyl or thiazolyl,
or R^{1'} denotes 5-methyl-2-oxo-1,3-dioxol-4-yl-methyl
and
R^{2'} independently of R^{1'} is hydrogen or R^{1'}, it also being possible for R^{2'} to be identical to R^{1'},
and
X' denotes oxygen or R^{3'}-substituted nitrogen, in which R^{3'} is hydrogen or C₁-C₃-alkyl or optionally together with R^{1'} forms a heterocyclic, saturated 6-membered ring, it also being possible for the heterocyclic 6-membered ring to include a second nitrogen atom and in turn to be substituted by phenyl or phenyl-C₁-C₃-alkyl,
or a physiologically tolerated salt thereof, excluding the compounds in which X' denotes oxygen and R^{1'} and R^{2'} are methyl or ethyl which is substituted by chlorine, hydroxyl or phenyl, and di(4-methoxyphenyl) pyridine-2,5-dicarboxylate.

5. The process as claimed in claim 3 or 4, wherein, in process variant a), the reaction is carried out with simultaneous addition of a dialkylcarbodiimide, the dialkyl radicals containing 1 to 8 carbon atoms and it also being possible, in the case of the C₃-C₈ compounds, for these to be branched or cyclic.

6. A process for the preparation of a pharmaceutical for inhibiting proline hydroxylase and lysine hydroxylase, which comprises bringing a compound of the formula I and I' as in claim 1 or 3 into a suitable administration form.

7. A process for the preparation of a pharmaceutical for use as a fibrosuppressant or immunosuppressant, which comprises bringing a compound of the formula I or I' as in claim 1 or 3 into a suitable administration form.

8. A process for the preparation of a pharmaceutical for influencing the metabolism of collagen and collagen-like substances or the biosynthesis of C1_{q}, which comprises bringing a compound of the formula I or I' as claimed in claim 1 or 3 into a suitable administration form.

9. The process as claimed in claim 3 for the preparation of a pyridine-2,4- or -2,5-dicarboxylic acid derivative of the formula I' as in claim 3, in which R^{1'} is branched or unbranched C₁-C₄-alkyl, which is substituted by alkoxycarbonyl, the alkyl radicals containing 1 - 3 carbon atoms and it also being possible, in the case of the C₃-alkyl radicals, for these to be branched, and R^{2'} independently of R^{1'} is hydrogen or R^{1'}, it also being possible for R^{2'} to be identical to R^{1'}, and X' denotes oxygen, or a physiologically tolerated salt thereof.

10. The process as claimed in claim 3 for the preparation of a pyridine-2,4- or -2,5-dicarboxylic acid derivative of the formula I' as in claim 3, in which R^{1'} and R^{2'} are 1-isopropoxycarbonylethyl groups and X' denotes oxygen, or a physiologically tolerated salt thereof.

## Revendications (Revendications pour l'(les) Etat(s) contractant(s) suivant(s): AT, BE, CH, DE, FR, GB, IT, LI, LU, NL, SE)

1. Dérivés d'acides pyridine-2,4-dicarboxylique et pyridine-2,5-dicarboxylique de formule I 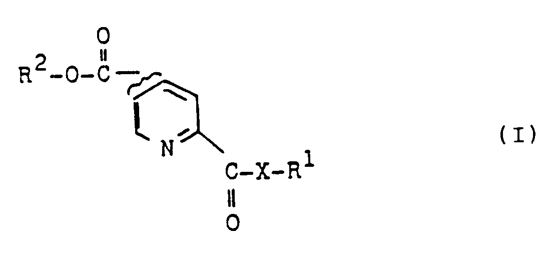 dans laquelle
R¹ représente un radical alkyle en C₁-C₁₂ ramifié ou non ramifié, qui est substitué une fois, ou, dans le cas des radicaux alkyle en C₂-C₁₂, également plusieurs fois, par
un ou des atomes d'halogène ou groupes hydroxy, cyano, carboxy, alcoxy, alcoxycarbonyle, alkylcarbonyloxy, alkyl- ou dialkylamino, les restes alkyle comportant de 1 à 4 atomes de carbone et, dans le cas des restes alkyle en C₃ et C₄, pouvant également être ramifiés, un groupe phényle qui, pour sa part, est éventuellement 1, 2 ou 3 fois substitué par un ou des atomes d'halogène ou radicaux nitro, alkyle en C₁-C₄ ou alcoxy en C₁-C₄, dans le cas de substitutions multiples les substituants pouvant également être différents indépendamment les uns des autres, et, dans le cas des radicaux alkyle en C₃-C₄, ceux-ci pouvant également être ramifiés,
ou R¹ est un radical cycloalkyle en C₅-C₇ saturé qui est éventuellement soudé à un noyau benzénique,
ou R¹ est un radical phényle ou naphtyle, ou un cycle aromatique à 5 ou 6 chaînons, comportant 1, 2 ou 3 atomes d'azote et/ou d'oxygène et/ou de soufre, qui pour leur part sont éventuellement 1, 2 ou 3 fois substitués par un ou des atomes d'halogène ou groupes alkyle en C₁-C₄ ou alcoxy en C₁-C₄, dans le cas de substitutions multiples les substituants pouvant également être différents indépendamment les uns des autres, et, dans le cas des groupes alkyle en C₃ et C₄, ceux-ci pouvant également être ramifiés,
ou R¹ est un radical 2-oxo-1,3-dioxolylméthyle qui est éventuellement encore substitué par le groupe méthyle,
ou R¹, lorsque X est un atome d'azote, est un atome d'hydrogène,
et
R² indépendamment de R¹ est un atome d'hydrogène ou a l'une des significations données pour R¹, R² pouvant également être identique à R¹,
et
X représente un atome d'oxygène ou un atome d'azote substitué par R³, R³ étant un atome d'hydrogène ou un groupe alkyle en C₁-C₆, et éventuellement forme conjointement avec R¹ un cycle hétérocyclique saturé à 5, 6 ou 7 chaînons, le cycle hétérocyclique pouvant également comporter un second atome d'azote, et le cycle hétérocyclique pouvant pour sa part être substitué par un groupe phényle ou phénylalkyle(C₁-C₃),
et sels physiologiquement acceptables de ceux-ci, pour utilisation en tant que médicament.

2. Dérivés d'acides pyridine-2,4-dicarboxylique et pyridine-2,5-dicarboxylique de formule I selon la revendication 1, dans lesquels
R¹ représente un radical alkyle en C₁-C₄ ramifié ou non ramifié, qui est substitué une fois, ou, dans le cas des radicaux alkyle en C₃ et C₄, également plusieurs fois, par un ou des groupes alcoxy en C₁-C₃ et/ou alcoxy(C₁-C₃)carbonyle, les restes alkyle en C₃ pouvant également être ramifiés, et/ou phényle,
ou R¹ représente un radical cycloalkyle en C₅ ou C₆ qui est éventuellement soudé à un noyau benzénique,
ou R¹ représente un radical phényle qui est éventuellement 1, 2 ou 3 fois substitué par le groupe nitro,
ou R¹ représente un radical 2-, 3- ou 4-pyridyle, naphtyle, 2- ou 3-thiényle, pyrazolyle, imidazolyle ou thiazolyle,
ou R¹ représente le radical 5-méthyl-2-oxo-1,3-dioxol-4-yl-méthyle et
R² indépendamment de R¹ est un atome d'hydrogène ou a l'une des significations données pour R¹, R² pouvant également être identique à R¹,
et
X représente un atome d'oxygène ou un atome d'azote substitué par R³, R³ étant un atome d'hydrogène ou un groupe alkyle en C₁-C₃, et éventuellement forme conjointement avec R¹ un cycle hétérocyclique saturé à 6 chaînons, le cycle hétérocyclique à 6 chaînons pouvant également comporter un second atome d'azote, et pouvant pour sa part être substitué par un groupe phényle ou phénylalkyle(C₁-C₃),
et sels physiologiquement acceptables de ceux-ci, pour utilisation en tant que médicament.

3. Dérivés d'acides pyridine-2,4-dicarboxylique et pyridine-2,5-dicarboxylique de formule I' 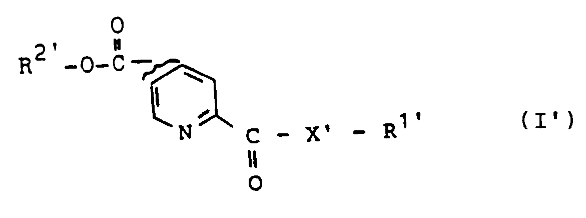 dans laquelle
R^{1'} représente un radical en C₁-C₁₂ ramifié ou non ramifié, qui est substitué une fois, ou, dans le cas des radicaux alkyle en C₂-C₁₂, également plusieurs fois, par un ou des atomes d'halogène ou groupes hydroxy, cyano, carboxy, alcoxy, alcoxycarbonyle, alkylcarbonyloxy, alkyl- ou dialkylamino, les restes alkyle comportant de 1 à 4 atomes de carbone, et, dans le cas des restes alkyle en C₃ et C₄, ceux-ci pouvant également être ramifiés, phényle, qui pour sa part est éventuellement 1, 2 ou 3 fois substitué par un ou des atomes d'halogène ou groupes alcoxy en C₁-C₄ ou alkyle en C₁-C₄, lesdits restes alkyle en C₃ et C₄ pouvant également être ramifiés et, dans le cas de substitutions multiples, les substituants pouvant également être différents indépendamment les uns des autres,
ou R^{1'} est un radical cycloalkyle en C₅-C₇ saturé qui est éventuellement soudé à un noyau benzénique,
ou R^{1'} est un radical phényle ou naphtyle, ou un cycle aromatique à 5 ou 6 chaînons, comportant 1, 2 ou 3 atomes d'azote et/ou d'oxygène et/ou de soufre, qui pour sa part est éventuellement 1, 2 ou 3 fois substitué par un ou des atomes d'halogène ou groupes nitro, alkyle en C₁-C₄ ou alcoxy en C₁-C₄, dans le cas de substitutions multiples les substituants pouvant également être différents indépendamment les uns des autres, et, dans le cas des groupes alkyle en C₃-C₄, ceux-ci pouvant également être être ramifiés,
ou R^{1'} est un groupe 2-oxo-1,3-dioxolylméthyle qui est éventuellement substitué par le groupe méthyle,
ou R^{1'}, lorsque X' est un atome d'azote, est un atome d'hydrogène,
et
R^{2'} indépendamment de R^{1'} est un atome d'hydrogène ou a l'une des significations données pour R^{1'}, R^{2'} pouvant également être identique à R^{1'},
et
X' représente un atome d'oxygène ou un atome d'azote substitué par R^{3'}, R^{3'} étant un atome d'hydrogène ou un groupe alkyle en C₁-C₆, et éventuellement forme conjointement avec R^{1'} un cycle hétérocyclique saturé à 5, 6 ou 7 chaînons, le cycle hétérocyclique pouvant également comporter un second atome d'azote, et le cycle hétérocyclique pouvant pour sa part être substitué par un groupe phényle ou phénylalkyle(C₁-C₃),
et sels physiologiquement acceptables de ceux-ci, à l'exclusion des composés dans lesquels X' représente un atome d'oxygène et R^{1'} et R^{2'} sont chacun un radical méthyle ou éthyle substitué par l'atome de chlore ou par le groupe hydroxy ou phényle, et du pyridine-2,5-dicarboxylate de di(4-méthoxyphényle).

4. Dérivés d'acides pyridine-2,4-carboxylique et pyridine-2,5-dicarboxylique de formule I' selon la revendication 3, dans lesquels
R^{1'} représente un radical alkyle en C₁-C₄ ramifié ou non ramifié, qui est substitué une fois, ou, dans le cas de radicaux alkyle en C₂-C₄, également plusieurs fois, par un ou des groupes alcoxy, alcoxycarbonyle, les restes alkyle comportant de 1 à 3 atomes de carbone et, dans le cas des radicaux alkyle en C₃, pouvant également être ramifiés, ou
phényle,
ou R^{1'} est un radical cyclopentyle ou cyclohexyle, éventuellement soudés à un noyau benzénique,
ou R^{1'} est un radical phényle qui est éventuellement substitué par 1, 2 ou 3 groupes nitro, ou est le radical naphtyle,
ou R^{1'} est le radical 2-, 3- ou 4-pyridyle, 3-thiényle, pyrazolyle, imidazolyle, thiazolyle
ou R^{1'} est le radical 5-méthyl-2-oxo-1,3-dioxol-4-yl-méthyle,
et
R^{2'} indépendamment de R^{1'} est un atome d'hydrogène ou a l'une des significations données pour R^{1'}, R^{2'} pouvant également être identique à R^{1'},
et
X' représente un atome d'oxygène ou un atome d'azote substitué par R^{3'}, R^{3'} étant un atome d'hydrogène ou un groupe alkyle en C₁-C₃, et éventuellement forme conjointement avec R^{1'} un cycle hétérocyclique saturé à 6 chaînons, le cycle hétérocyclique à 6 chaînons pouvant également comporter un second atome d'azote, et pouvant pour sa part être substitué par un groupe phényle ou phénylalkyle(C₁-C₃),
et sels physiologiquement acceptables de ceux-ci, à l'exclusion des composés dans lesquels X' représente un atome d'oxygène et R^{1'} et R^{2'} sont chacun un radical méthyle ou éthyle substitué par l'atome de chlore ou par le groupe hydroxy ou phényle, et du pyridine-2,5-dicarboxylate de di(4-méthoxyphényle).

5. Procédé pour la préparation de composés de formule I' selon la revendication 3, caractérisé en ce que
a) on fait réagir un composé de formule II 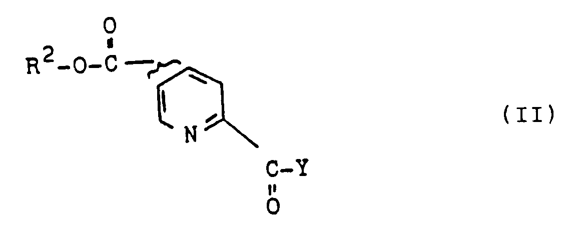 avec un composé de formule III
HX'-R^{1'} (III)
R^{1'}, R^{2'} et X' ayant les significations données à propos de la formule I' et Y étant un atome d'halogène ou le groupe hydroxy, ou
b) on fait réagir un composé de formule IV 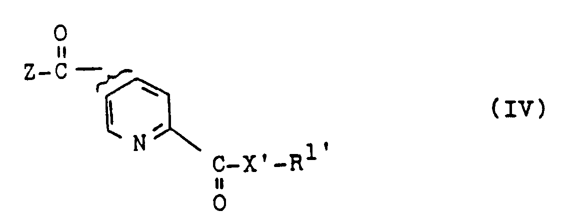 avec un composé de formule V
HO-R^{2'} (V)
R^{1'}, R^{2'} et X' ayant les significations données à propos de la formule I' et Z étant un atome d'halogène, ou
c) on fait réagir un composé de formule VI 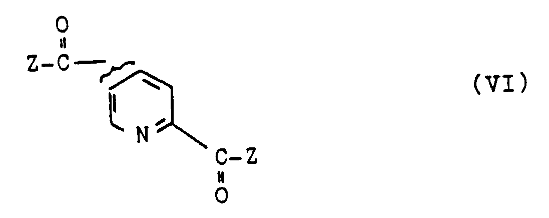 avec un alcool HO-R^{2'} ou avec un alcool de formule VII
HO-R^{1'} (VII)
R^{1'} et R^{2'} ayant les significations données à propos de la formule I' et Z étant un atome d'halogène, ou
d) on fait réagir un sel alcalin de l'acide pyridine-2,4- ou -2,5-dicarboxylique avec un halogénure de formule VIII
R^{1'}-Z (VIII)
R^{1'} ayant les significations données à propos de la formule I' et Z étant un atome d'halogène,
et éventuellement on convertit les produits de réaction en leurs sels physiologiquement acceptables.

6. Procédé selon la revendication 5, caractérisé en ce que, dans la variante a) du procédé, la réaction s'effectue avec addition simultanée d'un dialkylcarbodiimide, les restes dialkyle comportant de 1 à 8 atomes de carbone, et, dans le cas des radicaux en C₃-C₈, pouvant également être ramifiés ou cycliques.

7. Composés selon l'une des revendications 1 à 4, pour l'inhibition de la proline hydroxylase et de la lysine hydroxylase.

8. Composés selon l'une des revendications 1 à 4, pour utilisation en tant que fibrosuppresseurs et immunosuppresseurs.

9. Médicament contenant un composé de formule I ou I' selon la revendication 1 ou 3, avec des véhicules pharmaceutiquemes acceptables.

10. Procédé pour la fabrication de médicaments destinés à avoir un effet sur le métabolisme du collagène et de substances similaires au collagène, ou sur la biosynthèse du C1_{q}, caractérisé en ce que l'on incorpore dans le médicament un composé de formule I ou I' selon la revendication 1 ou 3.

11. Dérivés d'acides pyridine-2,4-dicarboxylique et pyridine-2,5-dicarboxylique de formule I' selon la revendication 3, dans lesquels R^{1'} représente un radical alkyle en C₁-C₄ ramifié ou non ramifié, qui est substitué par un groupe alcoxycarbonyle, les restes alkyle comportant de 1 à 3 atomes de carbone et, dans le cas des restes alkyle en C₃, pouvant également être ramifiés, et R^{2'} indépendamment de R^{1'} est un atome d'hydrogène ou a l'une des significations données pour R^{1'}, R^{2'} pouvant également être identique à R^{1'}, et X' représente un atome d'oxygène, et sels physiologiquement acceptables de ceux-ci.

12. Dérivés d'acides pyridine-2,4-dicarboxylique et d'acide pyridine-2,5-dicarboxylique de formule I' selon la revendication 3, dans lesquels R^{1'} et R^{2'} sont chacun le groupe 1-isopropoxycarbonyléthyle et X' représente un atome d'oxygène, et sels physiologiquement acceptables de ceux-ci.

## Revendications (Revendications pour l'(les) Etat(s) contractant(s) suivant(s): ES, GR)

1. Procédé pour la fabrication de médicaments, caractérisé en ce que l'on met sous une présentation appropriée des dérivés d'acides pyridine-2,4-dicarboxylique et pyridine-2,5-dicarboxylique de formule I 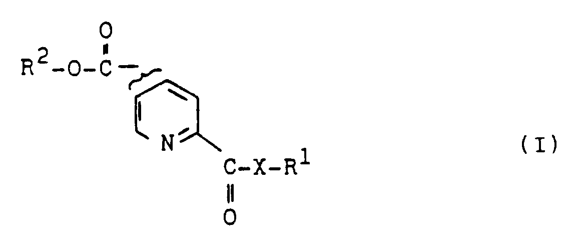 dans laquelle
R¹ représente un radical alkyle en C₁-C₁₂ ramifié ou non ramifié, qui est substitué une fois, ou, dans le cas des radicaux alkyle en C₂-C₁₂, également plusieurs fois, par
un ou des atomes d'halogène ou groupes hydroxy, cyano, carboxy, alcoxy, alcoxycarbonyle, alkylcarbonyloxy, alkyl- ou dialkylamino, les restes alkyle comportant de 1 à 4 atomes de carbone et, dans le cas des restes alkyle en C₃ et C₄, pouvant également être ramifiés, un groupe phényle qui, pour sa part, est éventuellement 1, 2 ou 3 fois substitué par un ou des atomes d'halogène ou radicaux nitro, alkyle en C₁-C₄ ou alcoxy en C₁-C₄, dans le cas de substitutions multiples les substituants pouvant également être différents indépendamment les uns des autres, et, dans le cas des radicaux alkyle en C₃-C₄, ceux-ci pouvant également être ramifiés,
ou R¹ est un radical cycloalkyle en C₅-C₇ saturé qui est éventuellement soudé à un noyau benzénique,
ou R¹ est un radical phényle ou naphtyle, ou un cycle aromatique à 5 ou 6 chaînons, comportant 1, 2 ou 3 atomes d'azote et/ou d'oxygène et/ou de soufre, qui pour leur part sont éventuellement 1, 2 ou 3 fois substitués par un ou des atomes d'halogène ou groupes alkyle en C₁-C₄ ou alcoxy en C₁-C₄, dans le cas de substitutions multiples les substituants pouvant également être différents indépendamment les uns des autres, et, dans le cas des groupes alkyle en C₃ et C₄, ceux-ci pouvant également être ramifiés,
ou R¹ est un radical 2-oxo-1,3-dioxolylméthyle qui est éventuellement encore substitué par le groupe méthyle,
ou R¹, lorsque X est un atome d'azote, est un atome d'hydrogène,
et
R² indépendamment de R¹ est un atome d'hydrogène ou a l'une des significations données pour R¹, R² pouvant également être identique à R¹,
et
X représente un atome d'oxygène ou un atome d'azote substitué par R³, R³ étant un atome d'hydrogène ou un groupe alkyle en C₁-C₆, et éventuellement forme conjointement avec R¹ un cycle hétérocyclique saturé à 5, 6 ou 7 chaînons, le cycle hétérocyclique pouvant également comporter un second atome d'azote, et le cycle hétérocyclique pouvant pour sa part être substitué par un groupe phényle ou phénylalkyle(C₁-C₃),
et leurs sels physiologiquement acceptables.

2. Procédé pour la fabrication de médicaments, caractérisé en ce que l'on met sous une présentation appropriée des dérivés d'acides pyridine-2,4-dicarboxylique et pyridine-2,5-dicarboxylique de formule I selon la revendication 1, dans lesquels
R¹ représente un radical alkyle en C₁-C₄ ramifié ou non ramifié, qui est substitué une fois, ou, dans le cas des radicaux alkyle en C₃ et C₄, également plusieurs fois, par un ou des groupes alcoxy en C₁-C₃ et/ou alcoxy(C₁-C₃)carbonyle, les restes alkyle en C₃ pouvant également être ramifiés, et/ou phényle,
ou R¹ représente un radical cycloalkyle en C₅ ou C₆ qui est éventuellement soudé à un noyau benzénique,
ou R¹ représente un radical phényle qui est éventuellement 1, 2 ou 3 fois substitué par le groupe nitro,
ou R¹ représente un radical 2-, 3- ou 4-pyridyle, naphtyle, 2- ou 3-thiényle, pyrazolyle, imidazolyle ou thiazolyle,
ou R¹ représente le radical 5-méthyl-2-oxo-1,3-dioxol-4-yl-méthyle et
R² indépendamment de R¹ est un atome d'hydrogène ou a l'une des significations données pour R¹, R² pouvant également être identique à R¹,
et
X représente un atome d'oxygène ou un atome d'azote substitué par R³, R³ étant un atome d'hydrogène ou un groupe alkyle en C₁-C₃, et éventuellement forme conjointement avec R¹ un cycle hétérocyclique saturé à 6 chaînons, le cycle hétérocyclique à 6 chaînons pouvant également comporter un second atome d'azote, et pouvant pour sa part être substitué par un groupe phényle ou phénylalkyle(C₁-C₃),
et leurs sels physiologiquement acceptables.

3. Procédé pour la préparation de dérivés d'acides pyridine-2,4-dicarboxylique et pyridine-2,5-dicarboxylique de formule I' 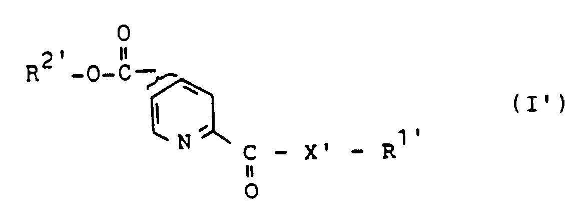 dans laquelle
R^{1'} représente un radical en C₁-C₁₂ ramifié ou non ramifié, qui est substitué une fois, ou, dans le cas des radicaux alkyle en C₂-C₁₂, également plusieurs fois, par un ou des atomes d'halogène ou groupes hydroxy, cyano, carboxy, alcoxy, alcoxycarbonyle, alkylcarbonyloxy, alkyl- ou dialkylamino, les restes alkyle comportant de 1 à 4 atomes de carbone, et, dans le cas des restes alkyle en C₃ et C₄, ceux-ci pouvant également être ramifiés, phényle, qui pour sa part est éventuellement 1, 2 ou 3 fois substitué par un ou des atomes d'halogène ou groupes alcoxy en C₁-C₄ ou alkyle en C₁-C₄, lesdits restes alkyle en C₃ et C₄ pouvant également être ramifiés et, dans le cas de substitutions multiples, les substituants pouvant également être différents indépendamment les uns des autres,
ou R^{1'} est un radical cycloalkyle en C₅-C₇ saturé qui est éventuellement soudé à un noyau benzénique,
ou R^{1'} est un radical phényle ou naphtyle, ou un cycle aromatique à 5 ou 6 chaînons, comportant 1, 2 ou 3 atomes d'azote et/ou d'oxygène et/ou de soufre, qui pour sa part est éventuellement 1, 2 ou 3 fois substitué par un ou des atomes d'halogène ou groupes nitro, alkyle en C₁-C₄ ou alcoxy en C₁-C₄, dans le cas de substitutions multiples les substituants pouvant également être différents indépendamment les uns des autres, et, dans le cas des groupes alkyle en C₃-C₄, ceux-ci pouvant également être être ramifiés,
ou R^{1'} est un groupe 2-oxo-1,3-dioxolylméthyle qui est éventuellement substitué par le groupe méthyle,
ou R^{1'}, lorsque X' est un atome d'azote, est un atome d'hydrogène,
et
R^{2'} indépendamment de R^{1'} est un atome d'hydrogène ou a l'une des significations données pour R^{1'}, R^{2'} pouvant également être identique à R^{1'},
et
X' représente un atome d'oxygène ou un atome d'azote substitué par R^{3'}, R^{3'} étant un atome d'hydrogène ou un groupe alkyle en C₁-C₆, et éventuellement forme conjointement avec R^{1'} un cycle hétérocyclique saturé à 5, 6 ou 7 chaînons, le cycle hétérocyclique pouvant également comporter un second atome d'azote, et le cycle hétérocyclique pouvant pour sa part être substitué par un groupe phényle ou phénylalkyle(C₁-C₃),
et de leurs sels physiologiquement acceptables, à l'exclusion des composés dans lesquels X' représente un atome d'oxygène et R^{1'} et R^{2'} sont chacun un radical méthyle ou éthyle substitué par l'atome de chlore ou par le groupe hydroxy ou phényle, et du pyridine-2,5-dicarboxylate de di(4-méthoxyphényle), caractérisé en ce que
a) on fait réagir un composé de formule II 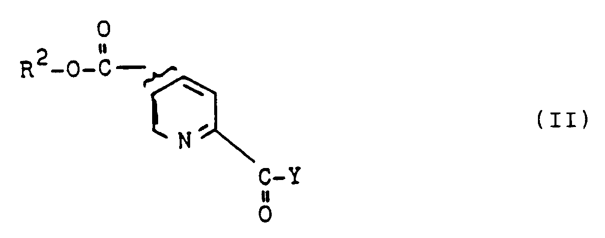 avec un composé de formule III
HX'-R^{1'} (III)
R^{1'}, R^{2'} et X' ayant les significations données à propos de la formule I' et Y étant un atome d'halogène ou le groupe hydroxy, ou
b) on fait réagir un composé de formule IV 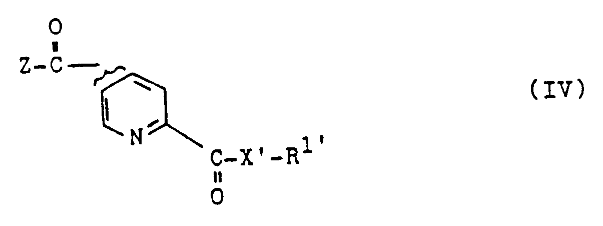 avec un composé de formule V
HO-R^{2'} (V)
R^{1'}, R^{2'} et X' ayant les significations données à propos de la formule I' et Z étant un atome d'halogène, ou
c) on fait réagir un composé de formule VI 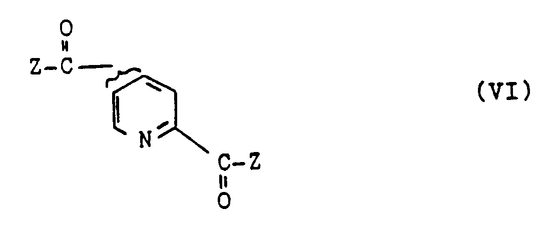 avec un alcool HO-R^{2'} ou avec un alcool de formule VII
HO-R^{1'} (VII)
R^{1'} et R^{2'} ayant les significations données à propos de la formule I' et Z étant un atome d'halogène, ou
d) on fait réagir un sel alcalin de l'acide pyridine-2,4- ou -2,5-dicarboxylique avec un halogénure de formule VIII
R^{1'}-Z (VIII)
R^{1'} ayant les significations données à propos de la formule I' et Z étant un atome d'halogène,
et éventuellement on convertit les produits de réaction en leurs sels physiologiquement acceptables.

4. Procédé selon la revendication 3, pour la préparation de dérivés d'acides pyridine-2,4-carboxylique et pyridine-2,5-dicarboxylique de formule I', dans lesquels
R^{1'} représente un radical alkyle en C₁-C₄ ramifié ou non ramifié, qui est substitué une fois, ou, dans le cas de radicaux alkyle en C₂-C₄, également plusieurs fois, par un ou des groupes alcoxy, alcoxycarbonyle, les restes alkyle comportant de 1 à 3 atomes de carbone et, dans le cas des radicaux alkyle en C₃, pouvant également être ramifiés, ou
phényle,
ou R^{1'} est un radical cyclopentyle ou cyclohexyle, éventuellement soudés à un noyau benzénique,
ou R^{1'} est un radical phényle qui est éventuellement substitué par 1, 2 ou 3 groupes nitro, ou est le radical naphtyle,
ou R^{1'} est le radical 2-, 3- ou 4-pyridyle, 3-thiényle, pyrazolyle, imidazolyle, thiazolyle
ou R^{1'} est le radical 5-méthyl-2-oxo-1,3-dioxol-4-yl-méthyle,
et
R^{2'} indépendamment de R^{1'} est un atome d'hydrogène ou a l'une des significations données pour R^{1'}, R^{2'} pouvant également être identique à R^{1'},
et
X' représente un atome d'oxygène ou un atome d'azote substitué par R^{3'}, R^{3'} étant un atome d'hydrogène ou un groupe alkyle en C₁-C₃, et éventuellement forme conjointement avec R^{1'} un cycle hétérocyclique saturé à 6 chaînons, le cycle hétérocyclique à 6 chaînons pouvant également comporter un second atome d'azote, et pouvant pour sa part être substitué par un groupe phényle ou phénylalkyle-(C₁-C₃),
et de leurs sels physiologiquement acceptables, à l'exclusion des composés dans lesquels X' représente un atome d'oxygène et R^{1'} et R^{2'} sont chacun un radical méthyle ou éthyle substitué par l'atome de chlore ou par le groupe hydroxy ou phényle, et du pyridine-2,5-dicarboxylate de di(4-méthoxyphényle).

5. Procédé selon la revendication 3 ou 4, caractérisé en ce que, dans la variante a) du procédé, la réaction s'effectue avec addition simultanée d'un dialkylcarbodiimide, les restes dialkyle comportant de 1 à 8 atomes de carbone, et, dans le cas des radicaux en C₃-C₈, pouvant également être ramifiés ou cycliques.

6. Procédé pour la fabrication de médicaments destinés à l'inhibition de la proline hydroxylase et de la lysine hydroxylase, caractérisé en ce que l'on met sous une présentation appropriée des composés de formule I ou I' selon la revendication 1 ou 3.

7. Procédé pour la fabrication de médicaments pour utilisation en tant que fibrosuppresseurs et immunosuppresseurs, caractérisé en ce que l'on met sous une présentation appropriée des composés de formule I ou I' selon la revendication 1 ou 3.

8. Procédé pour la fabrication de médicaments destinés à avoir un effet sur le métabolisme du collagène et de substances apparentées au collagène, ou sur la biosynthèse du C1_{q}, caractérisé en ce que l'on met sous une présentation appropriée un composé de formule I ou I' selon la revendication 1 ou 3.

9. Procédé selon la revendication 3, pour la préparation de dérivés d'acides pyridine-2,4-dicarboxylique et pyridine-2,5-dicarboxylique de formule I' selon la revendication 3, dans lesquels R^{1'} représente un radical alkyle en C₁-C₄ ramifié ou non ramifié, qui est substitué par un groupe alcoxycarbonyle, les restes alkyle comportant de 1 à 3 atomes de carbone, et R^{2'} indépendamment de R^{1'} est un atome d'hydrogène ou a l'une des significations données pour R^{1'}, R^{2'} pouvant également être identique à R^{1'}, et X' représente un atome d'oxygène, et de leurs sels physiologiquement acceptables.

10. Procédé selon la revendication 3, pour la préparation de dérivés d'acides pyridine-2,4-dicarboxylique et d'acide pyridine-2,5-dicarboxylique de formule I' selon la revendication 3, dans lesquels R^{1'} et R^{2'} sont chacun le groupe 1-isopropoxycarbonyléthyle et X' représente un atome d'oxygène, et de leurs sels physiologiquement acceptables.
